# EUROPÄISCHE PATENTANMELDUNG

(11) **EP 2 208 778 A1**
(43) Veröffentlichungstag der Anmeldung: **21.07.2010**
(21) Anmeldenummer: 09000698.2
(22) Anmeldetag: 20.01.2009
(51) Int. Cl.: C12M 1/42, C12M 3/00

(54) **Verfahren und Vorrichtung zur elektrischen Behandlung von Reaktionsräumen**

(71) Anmelder: Lonza Cologne AG, 50829 Köln (DE)
(72) Erfinder: Altrogge, Ludger, 53894 Mechernich (DE); Gleissner, Timo, 53879 Euskirchen (DE); Heinze, Andreas, 50739 Köln (DE); Müller-Hartmann, Herbert, 50937 Köln (DE); Wirth, Andreas, 49525 Lengerich (DE)

(57) **Zusammenfassung**

Die Erfindung betrifft ein Verfahren zur Beaufschlagung mindestens eines Reaktionsraums mit mindestens einem elektrischen Spannungsimpuls durch Entladen mindestens einer Ladungsspeichereinrichtung zum Speichern elektrischer Ladungen. Erfindungsgemäß umfasst das Verfahren das Beaufschlagen eines Reaktionsraums mit mindestens einem Spannungsimpuls durch zumindest partielles Entladen einer zweiten Ladungsspeichereinrichtung und gleichzeitig Laden oder teilweises Entladen einer ersten Ladungsspeichereinrichtung. Die Erfindung betrifft ferner eine Vorrichtung 1 zur Beaufschlagung mindestens eines Reaktionsraums mit mindestens einem Spannungsimpuls, welche mindestens zwei elektrische Ladungsspeichereinrichtungen 10, 11 zum Speichern von elektrischen Ladungen und mindestens eine Spannungsversorgungseinrichtung 12 zum Laden der Ladungsspeichereinrichtungen 10, 11 und/oder mindestens eine Entladevorrichtung zum teilweisen Entladen der Ladungsspeichereinrichtungen 10,11 umfasst. Erfindungsgemäß ist vorgesehen, dass zumindest zwei der Ladungsspeichereinrichtungen 10, 11 ein gemeinsames Speichermodul bilden, das zu jeder Zeit entladbar ist.

## Beschreibung

Die Erfindung betrifft ein Verfahren zur Beaufschlagung mindestens eines Reaktionsraums mit mindestens einem elektrischen Spannungsimpuls durch Entladen mindestens einer Ladungsspeichereinrichtung zum Speichern elektrischer Ladungen. Die Erfindung betrifft ferner eine Vorrichtung zur Beaufschlagung mindestens eines Reaktionsraums mit mindestens einem Spannungsimpuls, welche mindestens zwei elektrische Ladungsspeichereinrichtungen zum Speichern von elektrischen Ladungen und mindestens eine Spannungsversorgungseinrichtung zum Laden der Ladungsspeichereinrichtungen und/oder mindestens eine Entladevorrichtung zum teilweisen Entladen der Ladungsspeichereinrichtungen umfasst.

Das Einbringen biologisch aktiver Moleküle, wie beispielsweise DNA, RNA oder Proteine, in lebende Zellen stellt ein wichtiges Instrument zur Untersuchung biologischer Funktionen dieser Moleküle dar. Eine bevorzugte Methode zum Einbringen von Fremdmolekülen in die Zellen ist dabei die Elektroporation, welche im Gegensatz zu chemischen Methoden nicht auf den gleichzeitigen Transport anderer biologisch aktiver Moleküle angewiesen ist. Bei der Elektroporation werden die Fremdmoleküle aus einer an die Zellen angepassten Pufferlösung oder einem Zellkulturmedium durch einen kurzzeitigen Stromfluss in die Zellen eingebracht, wobei durch die Wirkung der elektrischen Spannungsimpulse bzw. des dadurch entstehenden elektrischen Feldes und Stromflusses die Zellmembran für die Fremdmoleküle durchlässig gemacht wird. Die Zellsuspension befindet sich dabei häufig in einer sogenannten Küvette, d.h. einem schmalen, offenen Gefäß, dessen Probenraum zwei gegenüberliegende, parallele Elektroden in den Seitenwänden aufweist, welche zum Anlegen der elektrischen Spannung dienen. Durch die kurzzeitig entstehenden "Poren" in der Zellmembran gelangen die biologisch aktiven Moleküle zunächst in das Zytoplasma, in dem sie ggf. bereits ihre zu untersuchende Funktion ausüben können, und daraufhin unter bestimmten Bedingungen auch in den Zellkern. Durch das kurzzeitige Anlegen eines starken elektrischen Feldes, d.h. eines kurzen Spannungsimpulses mit hoher Stromdichte, können darüber hinaus auch Zellen, Zellderivate, subzelluläre Partikel und/oder Vesikel fusioniert werden. Bei dieser sogenannten Elektrofusion werden die Zellen beispielsweise zunächst durch ein inhomogenes elektrisches Wechselfeld in engen Membrankontakt gebracht. Durch das anschließende Anlegen eines elektrischen Feldimpulses kommt es dann zur Interaktion von Membranteilen, die schließlich zur Fusion führt. Für die Elektrofusion können dabei vergleichbare apparative Vorrichtungen verwendet werden, wie für die Elektroporation. Darüber hinaus können lebende Zellen durch elektrische Felder auch in einer ihre Eigenschaften verändernden Weise stimuliert werden.

Behältnisse mit mehreren Reaktionsräumen für die Elektroporation, Elektrofusion oder Elektrostimulation von lebenden Zellen, sowie alle Anwendungen, bei denen der Reaktionsansatz einem elektrischen Feld sowie einem elektrischen Strom ausgesetzt werden muss, werden vor allem bei biochemischen und pharmazeutischen Anwendungen verwendet, wenn eine Vielzahl von Reaktionsansätzen gleichzeitig getestet werden muss. Dabei ist das Bestreben eine möglichst große Anzahl von Reaktionsräumen, beispielsweise 96 oder 384, zusammengefasst in einem Behältnis zur Verfügung zu stellen insbesondere bei HT-Analysen (HT = high throughput) zu erkennen, da hier eine Vielzahl von Proben in möglichst kurzer Zeit getestet werden soll. Bei derartigen Behältnissen spricht man in der Regel von Mehrlochplatten, Mikrotiterplatten oder Multiwells. Die Behältnisse bestehen üblicherweise aus mehreren Reaktionsräumen, die jeweils zwei Elektroden aufweisen, welche mit dem Reaktionsansatz, beispielsweise einer Zellsuspension, im Reaktionsraum in Kontakt stehen. Die beiden Elektroden eines Reaktionsraums erzeugen bei Anlegen einer elektrischen Spannung im Inneren des Reaktionsraums ein elektrisches Feld. Bei elektrisch leitenden Substanzen entsteht durch die umgebenden Elektroden unterschiedlicher Polarität neben dem elektrischen Feld ein elektrischer Strom. Die Elektroden gleicher Polarität, d. h. beispielsweise alle Kathoden und/oder alle Anoden, verschiedener Reaktionsräume sind dabei entweder einstückig ausgebildet oder elektrisch miteinander gekoppelt, so dass sie über einen gemeinsamen elektrischen Kontakt mit der Spannungsquelle verbunden werden können.

Ein Problem bei der elektrischen Behandlung solcher Mikrotiterplatten besteht darin, dass die Zeit, die für die Beaufschlagung der einzelnen Reaktionsräume mit einem oder mehreren Spannungsimpuls(en) benötigt wird, sehr lang ist. Dies liegt daran, dass insbesondere bei Einzelprozessierung der Reaktionsgefäße sehr viele Spannungsimpulse nacheinander abgegeben werden müssen, was insbesondere bei hohen Spannungswerten und/oder Energien der elektrischen Impulse sehr hohe Anforderungen an die Kapazitäten und Ladungszeiten der verwendeten Ladungsspeicher bzw. Kondensatoren stellt.

Verfahren und Vorrichtungen zur Beaufschlagung von Reaktionsräumen mit mehreren elektrischen Spannungsimpulsen sind bekannt. So offenbart beispielsweise die US-A-6 150 148 ein Verfahren und eine Vorrichtung für die Elektroporation, wobei mehrere Spannungsimpulse zur Beaufschlagung eines Reaktionsraums erzeugt werden. Die Vorrichtung weist eine Spannungsquelle auf, mittels der zwei Kondensatoren über einen Spannungsteiler und jeweils einen Transistor geladen werden können. Die Kondensatoren werden über steuerbare Schalter entladen, so dass der Reaktionsraum mit mehreren aufeinander folgenden Spannungsimpulsen beaufschlagt werden kann, beispielsweise mindestens einem Spannungsimpuls mit relativ hoher Spannungsamplitude und mindestens einem Spannungsimpuls mit geringerer Spannungsamplitude. Bei der aufeinander folgenden Beaufschlagung mehrerer Reaktionsräume mit solchen Spannungsimpulsfolgen hat diese bekannte Vorrichtung aber den Nachteil, dass die beiden Kondensatoren nach jeder Behandlung eines Reaktionsraums, oder zumindest nach der Behandlung weniger Reaktionsräume, wieder aufgeladen werden müssen, so dass immer wieder Pausen entstehen, die die Prozesszeit insgesamt verlängern.

Aus der US-A-6 010 613 sind ferner ein Verfahren und eine Vorrichtung zur Erzeugung einer Sequenz von Spannungsimpulsen bekannt. Die Spannungsimpulse werden hier durch das nahezu vollständige Entladen eines einzelnen Kondensators erzeugt, der eine Kapazität aufweist, die zur Erzeugung einer Impulssequenz ausreicht. Zur Erzeugung des nächsten Impulses bzw. der nächsten Impulssequenz muss der Kondensator daher erst wieder auf die benötigte Spannungsamplitude aufgeladen werden. Die hierfür benötigte Zeit wirkt sich bei der Behandlung vieler Reaktionsräume negativ auf die gesamte Prozessdauer aus.

Der Erfindung liegt die Aufgabe zugrunde ein Verfahren und eine Vorrichtung zur Verfügung zu stellen, mit denen Reaktionsräume innerhalb möglichst kurzer Zeit mit jeweils einem oder mehreren Spannungsimpuls(en) beaufschlagt werden können.

Erfindungsgemäß wird die Aufgabe durch ein Verfahren zur Beaufschlagung mindestens eines Reaktionsraums mit mindestens einem elektrischen Spannungsimpuls durch zumindest partielles Entladen mindestens einer Ladungsspeichereinrichtung zum Speichern elektrischer Ladungen gelöst, das die folgenden Schritte umfasst:
a) Beaufschlagen mindestens eines Reaktionsraums mit mindestens einem Spannungsimpuls durch zumindest partielles Entladen einer ersten Ladungsspeichereinrichtung und gleichzeitig Laden oder teilweises Entladen einer zweiten Ladungsspeichereinrichtung;
b) Beaufschlagen desselben Reaktionsraums oder eines weiteren Reaktionsraums mit mindestens einem Spannungsimpuls durch zumindest partielles Entladen der zweiten Ladungsspeichereinrichtung und gleichzeitig Laden oder teilweises Entladen der während Schritt a) entladenen ersten Ladungsspeichereinrichtung.

Bei dem erfindungsgemäßen Verfahren geht durch das parallele bzw. gleichzeitige, zumindest partielle Auf- und Entladen der ersten bzw. zweiten Ladungsspeichereinrichtung zwischen den aufeinander folgenden elektrischen Behandlungen keine Zeit für das Aufladen der Ladungsspeichereinrichtungen verloren, so dass die gesamte Prozessdauer deutlich verringert wird. Durch das zyklische Entladen einer Ladungsspeichereinrichtung und das gleichzeitige Laden oder teilweises Entladen einer anderen Ladungsspeichereinrichtung steht immer mindestens eine geladene Ladungsspeichereinrichtung für den nächsten Entladevorgang zur Verfügung, so dass zwischen zwei Entladevorgängen keine zusätzliche Zeit für das Laden oder teilweise Entladen einer Ladungsspeichereinrichtung benötigt wird, sofern diese kürzer andauern als der Entladevorgang. Das erfindungsgemäße Verfahren ermöglicht also sehr schnelle Spannungsimpulsabfolgen, sei es bei Anwendungen mit Mehrfachimpulsen oder bei Anwendungen, bei denen mehrere Reaktionsräume nacheinander und/oder gleichzeitig mit Spannungsimpulsen beaufschlagt werden müssen. Insbesondere bei Hochdurchsatzverfahren kann dadurch die Prozessdauer deutlich reduziert werden.

"Zumindest partielles Entladen" im Sinne der Erfindung bedeutet, dass die jeweilige Ladungsspeichereinrichtung bei der Abgabe eines Spannungsimpulses nicht vollständig entladen werden muss, sondern lediglich die Spannungsdifferenz abgibt, die für den Spannungsimpuls erforderlich ist. Es kann also nach dem Entladen noch eine Restspannung in der Ladungsspeichereinrichtung verbleiben.

"Gleichzeitig" im Sinne der Erfindung bedeutet, dass die Lade- bzw. Entladevorgänge zumindest teilweise synchron ablaufen, d. h. beide Vorgänge können entweder synchron oder zeitlich versetzt ablaufen, wobei im letzteren Fall ein Vorgang noch bzw. schon läuft, während der andere Vorgang schon beendet ist bzw. noch nicht begonnen hat. Da es sich bei der zumindest partiellen Entladung bei der Abgabe eines Spannungsimpulses beispielsweise um ein zeitlich sehr kurzes Ereignis handeln kann, wird die betreffende Ladungsspeichereinrichtung in diesem Fall während des "gleichzeitigen" Lade- oder teilweisen Entladevorgangs vor dem Beginn und/oder nach dem Ende der Entladung inaktiv sein, bis der länger andauernde Lade- oder teilweise Entladevorgang abgeschlossen ist.

"Laden oder teilweises Entladen" im Sinne der Erfindung bedeutet, dass die Ladungsspeichereinrichtung in die Bereitschaft versetzt wird, den nächsten bzw. folgenden Spannungsimpuls abzugeben. D. h., es erfolgt eine Anpassung des Spannungsniveaus in der Ladungsspeichereinrichtung an die für den nächsten Spannungsimpuls benötigte Spannung. Dies kann einerseits durch Aufladen der Ladungsspeichereinrichtung auf die für den nächsten Spannungsimpuls benötigte Spannung oder andererseits durch teilweises Entladen bis auf die für den nächsten Spannungsimpuls vorgesehene Spannung erfolgen. Im zweiten Fall wird das Spannungsniveau in der Ladungsspeichereinrichtung durch teilweises Entladen mittels einer Entladevorrichtung, beispielsweise über eine elektrische Last, vorzugsweise mittels eines Entladewiderstands, an das neue, niedrigere Spannungsniveau für den nächsten Reaktionsraum angepasst.

Dabei ist es besonders vorteilhaft, wenn alle Schritte bis zum Beenden des Verfahrens beliebig oft wiederholt werden und/oder wenn das Verfahren nach dem letzten Schritt beendet wird. Vorzugsweise wird das erfindungsgemäße Verfahren mehrfach wiederholt, d. h. es werden mehrere Zyklen der Verfahrensschritte durchlaufen, so dass beispielsweise sehr viele Reaktionsräume nacheinander elektrisch behandelt werden können, wobei das Verfahren erst beendet wird, wenn alle zu behandelnden Reaktionsräume mit mindestens einem Spannungsimpuls beaufschlagt wurden. Das erfindungsgemäße Verfahren kann also beispielsweise problemlos in Hochdurchsatzverfahren unter Verwendung von Multiwellplatten, vorzugsweise 96-well- oder 384-well-Platten, eingesetzt werden.

In vorteilhafter Ausgestaltung der Erfindung kann das Verfahren die folgenden Schritte umfassen:
a) Beaufschlagen von mindestens zwei Reaktionsräumen mit jeweils mindestens einem Spannungsimpuls durch zumindest partielles Entladen von zwei ersten Ladungsspeichereinrichtungen und gleichzeitig Laden oder teilweises Entladen von zwei zweiten Ladungsspeichereinrichtungen;
b) Beaufschlagen derselben Reaktionsräume oder von zwei weiteren Reaktionsräumen mit jeweils mindestens einem Spannungsimpuls durch zumindest partielles Entladen der beiden zweiten Ladungsspeichereinrichtungen und gleichzeitig Laden oder teilweises Entladen der beiden während Schritt a) zumindest partiell entladenen ersten Ladungsspeichereinrichtungen.

Das erfindungsgemäße Verfahren kann also zur Beaufschlagung eines einzelnen Reaktionsraums bzw. einer Küvette mit mehreren Spannungsimpulsen oder der elektrischen Behandlung mehrerer Reaktionsräume eingesetzt werden. Im letzteren Fall können nacheinander viele Reaktionsräume in kurzer Zeit mit Spannungsimpulsen beaufschlagt werden, wobei immer mindestens zwei Reaktionsräume gleichzeitig behandelt werden können. Bei dem erfindungsgemäßen Verfahren können folglich in vorteilhafter Ausgestaltung der Erfindung mindestens zwei Reaktionsräume gleichzeitig mit Spannungsimpulsen beaufschlagt werden, so dass sich die Geschwindigkeit der Behandlung von mehreren Reaktionsräumen deutlich erhöht.

In weiterer vorteilhafter Ausgestaltung der Erfindung kann das Verfahren ferner die folgenden Schritte umfassen:
a) Beaufschlagen mindestens eines Reaktionsraums mit mindestens einem Spannungsimpuls durch zumindest partielles Entladen einer ersten Ladungsspeichereinrichtung;
b) Beaufschlagen des Reaktionsraums mit mindestens einem weiteren Spannungsimpuls durch zumindest partielles Entladen einer zweiten Ladungsspeichereinrichtung und gleichzeitig Laden oder teilweises Entladen einer weiteren zweiten Ladungsspeichereinrichtung sowie der während Schritt a) entladenen ersten Ladungsspeichereinrichtung;
c) Beaufschlagen eines anderen oder desselben Reaktionsraums mit mindestens einem Spannungsimpuls durch zumindest partielles Entladen der während Schritt a) zumindest partiell entladenen und während Schritt b) wieder geladenen oder teilweise entladenen ersten Ladungsspeichereinrichtung;
d) Beaufschlagen des Reaktionsraums aus Schritt c) mit jeweils mindestens einem weiteren Spannungsimpuls durch zumindest partielles Entladen der während Schritt b) geladenen oder teilweise entladenen weiteren zweiten Ladungsspeichereinrichtung und gleichzeitig Laden oder teilweises Entladen der während Schritt b) zumindest partiell entladenen zweiten Ladungsspeichereinrichtung sowie der während Schritt c) zumindest partiell entladenen ersten Ladungsspeichereinrichtung.

Diese erfindungsgemäße Lösung ist insbesondere dann von Vorteil, wenn Spannungsimpulse erzeugt werden sollen, die sich aus einem kurzen Spannungsimpuls mit relativ hoher Spannungsamplitude und/oder relativ geringer Energie und einem längeren Spannungsimpuls mit geringerer Spannungsamplitude und/oder höherer Energie zusammensetzen. Sollen mehrere Reaktionsräume nacheinander in relativ kurzer Zeit mit derartigen Doppelspannungsimpulsen beaufschlagt werden, beispielsweise bei der Verwendung von Multiwellplatten im Hochdurchsatzverfahren, so wären die Abstände zwischen der Beaufschlagung der einzelnen Reaktionsräume bei der Verwendung eines einzelnen Kondensators für den längeren Spannungsimpuls relativ lang, da der Kondensator nach dem Entladen erst wieder durch Laden oder teilweises Entladen an die für den nächsten Spannungsimpuls vorgegebene Spannung angepasst werden müsste und hierfür größere Mengen elektrischer Energie aufzubringen sind. Bei dem erfindungsgemäßen Verfahren wird dagegen immer eine der beiden Ladungsspeichereinrichtungen entladen, während die andere Ladungsspeichereinrichtung geladen oder teilweise entladen wird, so dass sehr viele Reaktionsräume innerhalb kurzer Zeit mit Spannungsimpulsen beaufschlagt werden können. Das Aufladen oder teilweise Entladen der ersten Ladungsspeichereinrichtung mit geringerem Energiebedarf ist dabei kein zeitlich limitierender Faktor, da die erste Ladungsspeichereinrichtung, z.B. während der länger andauernden Entladung einer der beiden zweiten Ladungsspeichereinrichtungen, wieder aufgeladen oder teilweise entladen wird.

In besonders vorteilhafter Ausgestaltung des erfindungsgemäßen Verfahrens ist vorgesehen, dass das Verfahren die folgenden Schritte umfasst:
a) Beaufschlagen von mindestens zwei Reaktionsräumen mit jeweils mindestens einem Spannungsimpuls durch zumindest partielles Entladen von zwei ersten Ladungsspeichereinrichtungen;
b) Beaufschlagen der beiden Reaktionsräume aus Schritt a) mit jeweils mindestens einem weiteren Spannungsimpuls durch zumindest partielles Entladen von zwei zweiten Ladungsspeichereinrichtungen und gleichzeitig Laden oder teilweises Entladen von zwei weiteren zweiten Ladungsspeichereinrichtungen sowie der beiden während Schritt a) zumindest partiell entladenen ersten Ladungsspeichereinrichtungen;
c) Beaufschlagen derselben Reaktionsräume oder von zwei weiteren Reaktionsräumen mit jeweils mindestens einem Spannungsimpuls durch zumindest partielles Entladen der beiden während Schritt a) zumindest partiell entladenen und während Schritt b) wieder geladenen oder teilweises entladenen ersten Ladungsspeichereinrichtungen;
d) Beaufschlagen der beiden Reaktionsräume aus Schritt c) mit jeweils mindestens einem weiteren Spannungsimpuls durch zumindest partielles Entladen der beiden während Schritt b) geladenen oder teilweise entladenen weiteren zweiten Ladungsspeichereinrichtungen und gleichzeitig Laden oder teilweises Entladen der beiden während Schritt b) zumindest partiell entladenen zweiten Ladungsspeichereinrichtungen sowie der beiden während Schritt c) zumindest partiell entladenen ersten Ladungsspeichereinrichtungen.

Zu Beginn des erfindungsgemäßen Verfahrens werden vorzugsweise zunächst mindestens zwei erste Ladungsspeichereinrichtungen gleichzeitig oder nacheinander geladen. Nach Abschluss der Ladevorgänge werden dann diese zwei ersten Ladungsspeichereinrichtungen entladen, wobei mindestens zwei Reaktionsräume mit jeweils einem Spannungsimpuls, vorzugsweise einem kurzen Spannungsimpuls mit relativ hoher Spannungsamplitude, beaufschlagt werden. Diese beiden Reaktionsräume werden dann nach einer kurzen Pause oder ohne Unterbrechung jeweils mit einem weiteren Spannungsimpuls beaufschlagt, vorzugsweise einem längeren Spannungsimpuls mit geringerer Spannungsamplitude, wobei diese Spannungsimpulse durch die zumindest partielle Entladung der zweiten Ladungsspeichereinrichtungen erzeugt werden. Gleichzeitig mit der Entladung dieser zweiten Ladungsspeichereinrichtungen werden mindestens zwei weitere zweite Ladungsspeichereinrichtungen sowie die beiden ersten Ladungsspeichereinrichtungen geladen oder teilweise entladen, so dass die gesamte Prozessdauer durch die Lade- bzw. Entladevorgänge nicht verlängert wird und die entsprechenden Ladungsspeichereinrichtungen sofort wieder zur Verfügung stehen, wenn der nächste Reaktionsraum behandelt werden muss. Durch die anschließende Entladung der beiden ersten Ladungsspeichereinrichtungen können dann dieselben oder zwei weitere Reaktionsräume mit jeweils einem Spannungsimpuls beaufschlagt werden. Anschließend werden dann die zuvor geladenen oder teilweise entladenen zweiten Ladungsspeichereinrichtungen wieder zumindest partiell entladen, während zeitlich parallel die zuvor entladenen Ladungsspeichereinrichtungen wieder geladen oder teilweise entladen werden. Die zuvor beschriebenen Lade- und Entladevorgänge können beliebig oft wiederholt werden, so dass sehr viele Spannungsimpulse in relativ kurzer Zeit abgegeben werden können. Auf diese Weise kann auch eine Vielzahl von Reaktionsräumen in einer relativ kurzen Zeit mit Spannungsimpulsen, insbesondere Zweifachimpulsen, beaufschlagt werden.

In vorteilhafter Ausgestaltung der Erfindung können zu Beginn des Verfahrens vor Schritt a) alle Ladungsspeichereinrichtungen aufgeladen werden, so dass alle Ladungsspeichereinrichtungen bei Bedarf entladen werden können. Alternativ kann die jeweilige Ladungsspeichereinrichtung auch erst kurz vor dem Entladen aufgeladen werden.

Es ist besonders vorteilhaft, wenn jeweils mindestens zwei Ladungsspeichereinrichtungen ein Speichermodul bilden, wobei immer eine der Ladungsspeichereinrichtungen in der Anwendung zumindest partiell entladen wird oder zur Entladung bereit steht, während die andere Ladungsspeichereinrichtung geladen oder teilweise entladen wird, so dass das Speichermodul insgesamt zu jeder Zeit die für den nächsten Spannungsimpuls benötigte Spannung bereithält und zumindest partiell entladen werden kann. Die Lade- und Entladevorgänge können dabei entweder synchron oder zeitlich versetzt durchgeführt werden.

In vorteilhafter Ausgestaltung des Verfahrens ist vorgesehen, dass die ersten Ladungsspeichereinrichtungen eine Kapazität von mindestens 10 µF, vorzugsweise mindestens 50 µF, insbesondere mindestens 100 µF, aufweisen und/oder mit einer Anfangsspannung von 10 bis 1500 V, vorzugsweise 20 bis 1200 V, insbesondere 50 bis 1000 V, bei einer Pulsdauer von 2 µs bis 40 ms, vorzugsweise 5 µs bis 20 ms, insbesondere 10 µs bis 10 ms, entladen werden.

In vorteilhafter Ausgestaltung des Verfahrens ist ferner vorgesehen, dass die zweiten Ladungsspeichereinrichtungen eine Kapazität von mindestens 100 µF, vorzugsweise mindestens 500 µF, insbesondere mindestens 1000 µF, aufweisen und/oder mit einer Anfangsspannung von 10 bis 240 V, vorzugsweise 20 bis 200 V, insbesondere 50 bis 150 V, bei einer Pulsdauer von 1 ms bis 500 ms, vorzugsweise 2 ms bis 350 ms, insbesondere 4 ms bis 250 ms, entladen werden.

Insbesondere in Hochdurchsatzverfahren ist es vorteilhafterweise möglich, dass die Schritte c) und d) oder die Schritte b) bis e) mindestens 6-, 8-, 16-, 24-, 32-, 48-, 64-, 96-, 128-, 192-, 256-, 384- 1536-, 3456- oder 6144-mal wiederholt werden.

Dabei können Behältnisse, die mehrere Reaktionsräume umfassen, vorzugsweise 6, 8, 16, 24, 32, 48, 64, 96, 128, 192, 256, 384, 1536, 3456 oder 6144 in kurzer Zeit nacheinander mit jeweils mindestens einem Spannungsimpuls beaufschlagt werden.

Dabei ist es besonders von Vorteil, wenn Reaktionsräume nacheinander beaufschlagt werden, die räumlich möglichst weit voneinander entfernt sind, da auf diese Weise negative Effekte durch die durch die Spannungsimpulse verursachte Wärmeentwicklung besser verteilt werden können.

Es ist ferner von besonderem Vorteil, wenn das Entladen der Ladungsspeichereinrichtungen derart gesteuert wird, dass mindestens zwei Reaktionsräume nacheinander mit Spannungsimpulsen unterschiedlicher Spannungen beaufschlagt werden, bei denen die nach der Beaufschlagung des ersten Reaktionsraums in der jeweiligen Ladungsspeichereinrichtung verbleibende Spannung der Spannung des darauf folgend abzugebenden Spannungsimpulses möglichst nahe kommt. Dabei sollte die in der jeweiligen Ladungsspeichereinrichtung verbleibende Spannung idealer Weise etwas kleiner, etwas größer oder annähernd gleich der Spannung des darauf folgend abzugebenden Spannungsimpulses sein. Vorzugsweise sollte also eine so genannte Ladungshubminimierung erfolgen, d. h. dass jeweils ein Reaktionsraum für den nächsten Spannungsimpuls ausgewählt wird, der mit einer Spannungsamplitude beaufschlagt werden soll, die der in der Ladungsspeichereinrichtung verbleibenden Spannung am nächsten kommt, unabhängig davon, ob die folgende Spannung etwas höher oder etwas niedriger liegt. In jedem Fall sollten die zwischen den beiden Spannungsimpulsen nötigen Lade- oder Entladevorgänge minimiert werden. Vorzugsweise wird bei dieser Ausführungsform in aufsteigender Reihenfolge gepulst, d. h. die Spannung des ersten Spannungsimpulses ist geringer als die Spannung des folgenden Spannungsimpulses. Hierdurch können Entladevorgänge zwischen den Spannungsimpulsen vermieden werden. Durch das sequentielle Abarbeiten von Spannungsimpulsen kann die Prozessdauer in vorteilhafter Weise zusätzlich verringert werden.

Die Erfindung betrifft auch ein Verfahren zur Beaufschlagung von mindestens zwei Reaktionsräumen mit jeweils mindestens einem elektrischen Spannungsimpuls durch Entladen mindestens einer Ladungsspeichereinrichtung zum Speichern elektrischer Ladungen, bei dem das Entladen der Ladungsspeichereinrichtung derart gesteuert wird, dass mindestens zwei Reaktionsräume nacheinander mit Spannungsimpulsen unterschiedlicher Spannungen beaufschlagt werden, bei denen die nach der Beaufschlagung des ersten Reaktionsraums in der Ladungsspeichereinrichtung verbleibende Spannung der Spannung des darauf folgend abzugebenden Spannungsimpulses möglichst nahe kommt. Durch das erfindungsgemäße sequentielle Abarbeiten von Spannungsimpulsen kann die Prozessdauer in vorteilhafter Weise deutlich verringert werden, da zwischen zwei Spannungsimpulsen keine Zeit für Auf- oder Entladevorgänge benötigt bzw. diese Zeit zumindest deutlich reduziert wird. Vorzugsweise ist dabei die in der jeweiligen Ladungsspeichereinrichtung verbleibende Spannung etwas kleiner, etwas größer oder annähernd gleich der Spannung des darauf folgend abzugebenden Spannungsimpulses. Vorzugsweise sollte also eine sogenannte Ladungshubminimierung erfolgen, d. h. dass jeweils ein Reaktionsraum für den nächsten Spannungsimpuls ausgewählt wird, der mit einer Spannungsamplitude beaufschlagt werden soll, die der in der Ladungsspeichereinrichtung verbleibenden Spannung am nächsten kommt, unabhängig davon, ob die folgende Spannung etwas höher oder etwas niedriger liegt. In jedem Fall sollten die zwischen den beiden Spannungsimpulsen nötigen Lade- oder Entladevorgänge minimiert werden. In vorteilhafter Ausgestaltung der Erfindung wird dabei in aufsteigender Reihenfolge gepulst, d. h. die Spannung des ersten Spannungsimpulses ist geringer als die Spannung des folgenden Spannungsimpulses. Hierdurch können Entladevorgänge zwischen den Spannungsimpulsen vermieden werden.

Da die Ladungsspeichereinrichtung, z. B. die speisende Kondensatorbank, beim Pulsen Energie abgibt, reduziert sich die Spannung im Ladungsspeicher. Für den nächsten Reaktionsraum muss nun der Ladungsspeicher vor dem Pulsen wieder auf die für den nächsten Reaktionsraum erforderliche Spannung geladen werden. Werden die Spannungsimpulse innerhalb einer Kontaktiersektion beispielsweise nach abfallender Anfangsspannung sortiert, so kann der Lade- sowie Entladespannungshub minimiert und somit die Effizienz gesteigert werden.

Ferner kann die Reihenfolge in der die Reaktionsräume innerhalb einer Kontaktiersektion abgearbeitet werden, bei temperaturempfindlichen Experimenten so gewählt werden, dass die benachbarten Reaktionsräume innerhalb eines Behältnisses mit möglichst großem zeitlichen Abstand zueinander gepulst werden, um den Temperaturanstieg von einem Reaktionsraum neben einem gerade gepulsten Reaktionsraum möglichst schnell wieder abklingen zu lassen, bevor dieser Reaktionsraum mit einem Spannungsimpuls beaufschlagt wird.

Die Aufgabe wird ferner durch eine Vorrichtung gelöst, bei der zumindest zwei der Ladungsspeichereinrichtungen ein gemeinsames Speichermodul bilden, das zu jeder Zeit entladbar ist, so dass diese Ladungsspeichereinrichtungen eine Einheit bilden, die nach einem ersten Ladevorgang immer bei Bedarf entladen werden kann, ohne dass zwischendurch ein zeitraubender Auf- oder Entladevorgang durchgeführt werden muss.

Die mindestens zwei Ladungsspeichereinrichtungen sind vorzugsweise über eine gemeinsame Schnittstelle mit einer Impulsgenerierungseinheit, einer Schalteinheit und/oder einer Verteilvorrichtung für elektrische Spannungsimpulse elektrisch gekoppelt.

"Elektrisch gekoppelt" im Sinne der Erfindung bedeutet, dass zwei Komponenten bzw. Bauteile der elektrischen Schaltungsanordnung direkt, d. h. ohne zwischengeschaltete Bauteile, oder indirekt, d. h. mit zwischen die Komponenten geschaltete Bauteile, elektrisch miteinander verbunden sind.

Es kann ferner mindestens eine weitere Ladungsspeichereinrichtung vorgesehen sein, die vorzugsweise über mindestens eine weitere Schnittstelle mit der Impulsgenerierungseinheit, der Schalteinheit und/oder der Verteilvorrichtung für elektrische Spannungsimpulse elektrisch gekoppelt ist. Auf diese Weise ist es möglich, entweder mindestens zwei Reaktionsräume gleichzeitig zu behandeln oder aber Doppelimpulse zu erzeugen, mit denen mindestens ein Reaktionsraum beaufschlagt wird.

In diesen Fällen ist vorgesehen, dass die mindestens eine weitere Ladungsspeichereinrichtung mit einer Spannungsversorgungseinrichtung elektrisch gekoppelt ist und dass zumindest die mindestens zwei Ladungsspeichereinrichtungen mit einer gemeinsamen Spannungsversorgungseinrichtung elektrisch gekoppelt sind. Diese Ausführungsform ist insbesondere dann von Vorteil, wenn Spannungsimpulse erzeugt werden sollen, die sich aus einem kurzen Spannungsimpuls mit relativ hoher Spannungsamplitude und einem längeren Spannungsimpuls mit geringerer Spannungsamplitude zusammensetzen. In diesem Fall kann die Ladungsspeichereinrichtung einen kurzen Spannungsimpuls erzeugen, während das sich aus zwei Ladungsspeichereinrichtungen zusammensetzende Speichermodul einen längeren Spannungsimpuls zur Verfügung stellt. Bei der erfindungsgemäßen Vorrichtung kann folglich immer eine der beiden Ladungsspeichereinrichtungen eines Speichermoduls entladen werden, während die andere Ladungsspeichereinrichtung des Speichermoduls geladen wird, so dass sehr viele Reaktionsräume innerhalb kurzer Zeit mit Spannungsimpulsen beaufschlagt werden können. Das Aufladen der einzelnen Ladungsspeichereinrichtungen ist dabei kein zeitlich limitierender Faktor, da diese Ladungsspeichereinrichtung während der länger andauernden Entladung des Speichermoduls aufgeladen werden kann. Selbst für den Fall, dass das Spannungsversorgungsgerät (Netzteil) nicht stark und schnell genug sein sollte, kann durch das erfindungsgemäße Verfahren die für den Ladevorgang benötigte Zeit minimiert werden.

In besonders vorteilhafter Ausgestaltung der erfindungsgemäßen Vorrichtung können auch mindestens vier Ladungsspeichereinrichtungen vorgesehen sein, die insgesamt mindestens zwei Speichermodule bilden.

Es ist insbesondere von Vorteil, wenn ein Speichermodul über eine einzelne bzw. gemeinsame Schnittstelle mit der Impulsgenerierungseinheit, der Schalteinheit und/oder der Verteilvorrichtung für elektrische Spannungsimpulse elektrisch gekoppelt ist. Da beispielsweise vier Ladungsspeichereinrichtungen insgesamt zwei Speichermodule bilden können, die jeweils zu jeder Zeit entladbar sind, können durch die erfindungsgemäße Vorrichtung immer zwei Reaktionsräume gleichzeitig mit jeweils einem Spannungsimpuls beaufschlagt werden. Da zwischen zwei aufeinander folgenden Spannungsimpulsen keine Zeit für das Aufladen eines einzelnen Kondensators benötigt wird, können mittels der erfindungsgemäßen Vorrichtung folglich sehr viele Reaktionsräume in relativ kurzer Zeit mit Spannungsimpulsen beaufschlagt werden. Die erfindungsgemäße Vorrichtung eignet sich daher insbesondere für die Verwendung in Hochdurchsatzverfahren, bei denen sehr viele Reaktionsräume in kurzer Zeit mit jeweils mindestens einem Spannungsimpuls beaufschlagt werden müssen.

Es können erfindungsgemäß mindestens zwei Spannungsversorgungseinrichtungen vorgesehen sein, wobei jede Spannungsversorgungseinrichtung mit jeweils einem Speichermodul elektrisch gekoppelt ist.

Erfindungsgemäß können aber auch mindestens vier Spannungsversorgungseinrichtungen und mindestens sechs Ladungsspeichereinrichtungen vorgesehen sein. Diese Ausführungsform der erfindungsgemäßen Vorrichtung ist insbesondere in Hochdurchsatzverfahren besonders vorteilhaft einsetzbar, in denen viele Reaktionsräume in kurzer Zeit mit doppelten Spannungsimpulsen beaufschlagt werden müssen. Dabei können mittels der erfindungsgemäßen Vorrichtung immer zwei Reaktionsräume gleichzeitig mit Spannungsimpulsen beaufschlagt werden. So können beispielsweise zwei Ladungsspeichereinrichtungen gleichzeitig jeweils einen kurzen Spannungsimpuls mit relativ hoher Spannungsamplitude abgeben, der dann jeweils ohne Unterbrechung in einen längeren Spannungsimpuls mit geringerer Spannungsamplitude übergeht. Die längeren Spannungsimpulse können durch zwei Speichermodule zur Verfügung gestellt werden, wobei immer eine der beiden Ladungsspeichereinrichtungen eines Speichermoduls entladen wird.

Dabei können die Ladungsspeichereinrichtungen über mindestens vier Schnittstellen mit der Impulsgenerierungseinheit, der Schalteinheit und/oder der Verteilvorrichtung elektrisch gekoppelt sein.

Dabei können zumindest zwei der Ladungsspeichereinrichtungen mit jeweils einer Spannungsversorgungseinrichtung elektrisch gekoppelt und mindestens zwei Speichermodule vorgesehen sein, die jeweils mindestens zwei Ladungsspeichereinrichtungen umfassen, wobei zumindest zwei der Speichermodule jeweils mit einer gemeinsamen Spannungsversorgungseinrichtung elektrisch gekoppelt sind.
In besonders vorteilhafter Ausgestaltung der erfindungsgemäßen Vorrichtung ist vorgesehen, dass zumindest eine Ladungsspeichereinrichtung mit einer Impulsgenerierungseinheit elektrisch gekoppelt ist, welche ihrerseits, vorzugsweise über eine Schalteinheit, mit einer Verteilvorrichtung für elektrische Spannungsimpulse elektrisch gekoppelt ist.

In vorteilhafter Ausgestaltung der Erfindung ist vorgesehen, dass zumindest eine der Ladungsspeichereinrichtungen eine Kapazität von mindestens 10 µF, vorzugsweise mindestens 50 µF, insbesondere mindestens 100 µF, aufweist.

Zumindest eine der Ladungsspeichereinrichtungen kann aber auch eine Kapazität von mindestens 100 µF, vorzugsweise mindestens 500 µF, insbesondere mindestens 1000 µF, aufweisen.

Die Erfindung wird im Weiteren anhand der Figuren beispielhaft näher erläutert.

Die Figuren 1 bis 6 zeigen jeweils schematisch den zeitlichen Ablauf besonders vorteilhafter Ausführungsformen des erfindungsgemäßen Verfahrens (R = Reaktionsraum).

Die Figuren 7 bis 12 zeigen jeweils schematisch den Aufbau besonders vorteilhafter Ausführungsformen der erfindungsgemäßen Vorrichtung.

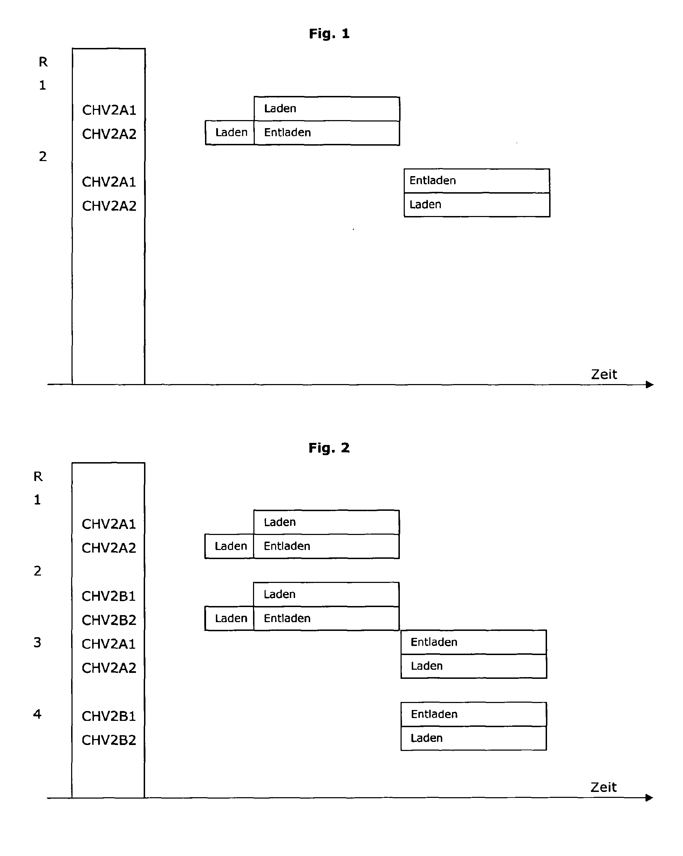

Figur 1 zeigt schematisch den zeitlichen Ablauf einer ersten besonders vorteilhaften Ausführungsform des erfindungsgemäßen Verfahrens. Dieses Ausführungsbeispiel eignet sich insbesondere zur Beaufschlagung mindestens eines Reaktionsraums mit mindestens einem Spannungsimpuls. Zu Beginn des Verfahrens wird hier zunächst die mit CHV2A2 bezeichnete Ladungsspeichereinrichtung geladen. Diese Ladungsspeichereinrichtung wird anschließend entladen, so dass mindestens ein erster Reaktionsraum R1 mit mindestens einem Spannungsimpuls beaufschlagt wird. Die zweite Ladungsspeichereinrichtung CHV2A1 wird parallel zur zumindest partiellen Entladung der ersten Ladungsspeichereinrichtung CHV2A2 geladen oder teilweise entladen. "Teilweise entladen" bedeutet, dass die Ladungsspeichereinrichtung in die Bereitschaft versetzt wird, den nächsten bzw. folgenden Spannungsimpuls abzugeben, d. h., es erfolgt eine Anpassung des Spannungsniveaus in der Ladungsspeichereinrichtung an die für den nächsten Spannungsimpuls benötigte Spannung. Dies kann entweder durch Aufladen der Ladungsspeichereinrichtung auf die für den nächsten Spannungsimpuls benötigte Spannung oder durch teilweises Entladen bis auf die für den nächsten Spannungsimpuls vorgesehene Spannung erfolgen. Im zweiten Fall wird das Spannungsniveau in der Ladungsspeichereinrichtung durch teilweises Entladen mittels einer Entladevorrichtung, beispielsweise über eine elektrische Last, vorzugsweise mittels eines Entladewiderstands, an das neue, niedrigere Spannungsniveau für den nächsten Reaktionsraum angepasst. Folglich steht die zweite Ladungsspeichereinrichtung CHV2A1 sofort für den nächsten Entladevorgang zur Verfügung, wenn die erste Ladungsspeichereinrichtung CHV2A2 entladen wurde. Der nächste Entladevorgang kann also ohne Pause unmittelbar nach dem ersten Entladevorgang beginnen, so dass zwischen zwei Spannungsimpulsen keine Zeit für das Laden oder das teilweise Entladen einer Ladungsspeichervorrichtung benötigt wird bzw. verloren geht. Unmittelbar nach dem zumindest partiellen Entladen der ersten Ladungsspeichereinrichtung CHV2A2 schließt sich im vorliegenden Ausführungsbeispiel demnach ohne Unterbrechung unmittelbar das Entladen der ersten Ladungsspeichereinrichtung CHV2A1 an. Durch diesen Entladevorgang wird im vorliegenden Ausführungsbeispiel ein weiterer Reaktionsraum R2 mit einem Spannungsimpuls beaufschlagt. Alternativ könnte aber auch der erste Reaktionsraum R1 mit einem weiteren Spannungsimpuls beaufschlagt werden. Da gleichzeitig zur Entladung der zweiten Ladungsspeichereinrichtung CHV2A1 die erste Ladungsspeichereinrichtung CHV2A2 wieder geladen oder teilweise entladen wird, kann das erfindungsgemäße Verfahren ohne Unterbrechung weiter fortgesetzt werden, so dass die selben oder weitere Reaktionsräume mit Spannungsimpulsen beaufschlagt werden können. Durch das gleichzeitige Entladen und Laden bzw. teilweises Entladen der Ladungsspeichereinrichtungen steht dabei immer eine Ladungsspeichereinrichtung zur Generierung eines Spannungsimpulses zur Verfügung, so dass die einzelnen Spannungsimpulse unmittelbar hintereinander abgegeben werden können. Wenn eine Vielzahl von Spannungsimpulsen hintereinander abgegeben werden muss, sei es für den Fall, dass ein Reaktionsraum mit einer Abfolge mehrerer Spannungsimpulse beaufschlagt werden muss, oder mehrere Reaktionsräume mit jeweils einem Spannungsimpuls beaufschlagt werden müssen, kann durch das erfindungsgemäße Verfahren folglich die gesamte Prozessdauer deutlich reduziert werden. Das erfindungsgemäße Verfahren eignet sich daher insbesondere für die Elektroporation, Elektrofusion oder ähnliche Verfahren im Durchfluss- oder Hochdurchsatzbetrieb. "Gleichzeitiges" Laden und Entladen der Ladungsspeichereinrichtungen bedeutet dabei, dass die Lade- und Entladevorgänge zumindest teilweise synchron ablaufen. Die Lade- und Entladevorgänge können also entweder parallel oder zeitlich versetzt ablaufen. "Zeitlich versetzt" bedeutet dabei, dass ein Vorgang noch bzw. schon läuft, während der andere Vorgang schon beendet ist bzw. noch nicht begonnen hat.

Figur 2 zeigt schematisch den zeitlichen Ablauf einer weiteren Ausführungsform des erfindungsgemäßen Verfahrens. Dieses Ausführungsbeispiel eignet sich insbesondere zur Beaufschlagung von mindestens zwei Reaktionsräumen mit jeweils mindestens einem Spannungsimpuls. Zu Beginn des Verfahrens werden zunächst die hier mit CHV2A2 und CHV2B2 bezeichneten Ladungsspeichereinrichtungen geladen. Diese ersten Ladungsspeichereinrichtungen werden anschließend zumindest partiell entladen, so dass mindestens zwei Reaktionsräume, hier R1 und R2, mit jeweils einem Spannungsimpuls beaufschlagt werden. Gleichzeitig zur Entladung der beiden ersten Ladungsspeichereinrichtungen werden die beiden zweiten Ladungsspeichereinrichtungen CHV2A1 und CHV2B1 geladen. "Gleichzeitig" im Sinne der Erfindung bedeutet, dass die Lade- bzw. Entladevorgänge zumindest teilweise synchron ablaufen, d. h. beide Vorgänge können synchron oder auch zeitlich versetzt ablaufen, wobei im letzteren Fall ein Vorgang noch bzw. schon läuft, während der andere Vorgang schon beendet ist bzw. noch nicht begonnen hat. Nach Abschluss dieser Be- und Entladevorgänge werden dann die zuvor geladenen zweiten Ladungsspeichereinrichtungen CHV2A1 und CHV2B1 entladen und gleichzeitig die zuvor entladenen ersten Ladungsspeichereinrichtungen CHV2A2 und CHV2B2 wieder aufgeladen oder teilweise entladen. Somit können die weiteren Reaktionsräume R3 und R4 jeweils mit einem Spannungsimpuls beaufschlagt werden, während bereits die nächsten Spannungsimpulse vorbereitet werden. Diese Schritte können beliebig oft wiederholt werden. Durch das gleichzeitige Be- und Entladen von mindestens zwei Ladungsspeichereinrichtungen können somit mindestens zwei Reaktionsräume gleichzeitig mit jeweils einem Spannungsimpuls beaufschlagt werden, wobei die nächsten beiden Reaktionsräume unmittelbar anschließend mit Spannungsimpulsen beaufschlagt werden können, ohne dass Zeit für das erneute Aufladen oder das teilweise Entladen der Ladungsspeichereinrichtungen verloren geht. Dies ist insbesondere dann von Vorteil, wenn eine Vielzahl von Reaktionsräumen mit Spannungsimpulsen beaufschlagt werden muss, da die Gesamtzeit, die zur Beaufschlagung aller Reaktionsräume mit Spannungsimpulsen aufgewendet werden muss, erheblich verkürzt wird.

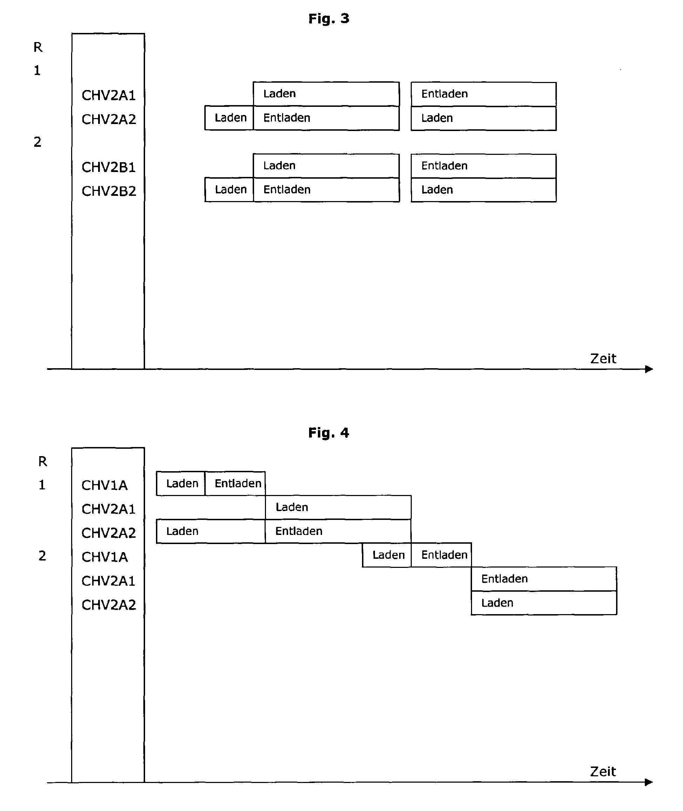

Figur 3 zeigt schematisch den zeitlichen Ablauf einer besonderen Ausführungsform des erfindungsgemäßen Verfahrens, die im Wesentlichen dem Verfahren gemäß Figur 2 entspricht. Im Unterschied zum Verfahren gemäß Figur 2 werden im vorliegenden Ausführungsbeispiel gemäß Figur 3 die selben Reaktionsräume R1 und R2 mit mehreren Spannungsimpulsen beaufschlagt. Während also bei der Ausführungsform gemäß Figur 2 nach den Reaktionsräumen R1 und R2 die weiteren Reaktionsräume R3 und R4 elektrisch behandelt werden, werden bei der Ausführungsform gemäß Figur 3 nur die Reaktionsräume R1 und R2 mit mehreren aufeinander folgenden Spannungsimpulsen beaufschlagt. Selbstverständlich ist es auch möglich die Ausführungsformen gemäß Figur 2 und Figur 3 zu kombinieren, so dass mehrere Reaktionsräume hintereinander jeweils mit mehreren Spannungsimpulsen beaufschlagt werden können.

Figur 4 zeigt schematisch den zeitlichen Ablauf einer besonders vorteilhaften Ausführungsform des erfindungsgemäßen Verfahrens. Diese Ausführungsform des erfindungsgemäßen Verfahrens eignet sich insbesondere zur Beaufschlagung mindestens eines Reaktionsraums mit mindestens zwei elektrischen Spannungsimpulsen. Zu Beginn des Verfahrens wird zunächst die mit CHV1A bezeichnete Ladungsspeichereinrichtung geladen. Diese Ladungsspeichereinrichtung wird dann zumindest partiell entladen, wobei der erste Reaktionsraum R1 mit einem Spannungsimpuls beaufschlagt wird. Zu irgendeinem Zeitpunkt während des Lade- bzw. Entladevorgangs der Ladungsspeichereinrichtung CHV1A wird eine zweite Ladungsspeichereinrichtung CHV2A2 geladen. Diese Ladungsspeichereinrichtung CHV2A2 wird unmittelbar nach der Entladung der ersten Ladungsspeichereinrichtung CHV1A zumindest partiell entladen, wobei hiermit ebenfalls der Reaktionsraum R1 mit dementsprechenden Spannungsimpuls beaufschlagt wird. Der Reaktionsraum R1 wird also nacheinander durch zwei aufeinander folgende Spannungsimpulse beaufschlagt, wobei diese beiden Spannungsimpulse vorzugsweise ohne Unterbrechung unmittelbar ineinander übergehen. In einer bevorzugten Ausführungsform der vorliegenden Erfindung kann es sich dabei bei dem ersten Spannungsimpuls um einen kurzen Impuls mit relativ hoher Spannungsamplitude und bei dem zweiten Spannungsimpuls um einen längeren Impuls mit geringerer Spannungsamplitude handeln. Die beiden Spannungsimpulse können alternativ auch nacheinander mit einer mehr oder weniger kurzen Pause abgesetzt werden, so dass ein Doppelimpuls mit Unterbrechung generiert wird. Gleichzeitig mit dem Entladen der zweiten Ladungsspeichereinrichtung CHV1A2 wird die weitere zweite Ladungsspeichereinrichtung CHV2A1 geladen. Während der Entladung der zweiten Ladungsspeichereinrichtung CHV2A2 werden die erste Ladungsspeichereinrichtung CHV1A und eine weitere zweite Ladungsspeichereinrichtung CHV2A1 geladen. Folglich stehen nach Abschluss der Entladung der zweiten Ladungsspeichereinrichtung CHV2A2 sofort die beiden Ladungsspeichereinrichtungen CHV1A und CHV2A1 wieder für die nächste Entladung zur Verfügung. Daher kann unmittelbar anschließend, ohne Pause der nächste Reaktionsraum R2 mit einem Doppelspannungsimpuls beaufschlagt werden. Dies geschieht zunächst durch zumindest partielles Entladen der ersten Ladungsspeichereinrichtung CHV1A und die direkt ohne Unterbrechung daran anschließende zumindest partielles Entladung der weiteren zweiten Ladungsspeichereinrichtung CHV2A1. Durch erneutes zumindest partielles Entladen der ersten Ladungsspeichereinrichtung CHV1A und der weiteren zweiten Ladungsspeichereinrichtung CHV2A1 kann alternativ auch der selbe Reaktionsraum R1 mit einem weiteren Spannungsimpuls beaufschlagt werden. Gleichzeitig mit der zumindest partiellen Entladung der weiteren zweiten Ladungsspeichereinrichtung CHV2A1 werden die erste Ladungsspeichereinrichtung CHV1A und die zweite Ladungsspeichereinrichtung CHV2A2 wieder geladen oder teilweise entladen, so dass der nächste Doppel-Spannungsimpuls ohne Verzögerung abgegeben werden kann. Die zuvor beschriebenen Lade- und Entladevorgänge können beliebig oft wiederholt werden, so dass sehr viele Spannungsimpulse in relativ kurzer Zeit abgeben werden können. Die Prozessdauer wird dabei auch bei der elektrischen Behandlung vieler Reaktionsräume minimiert, da zwischen den einzelnen Spannungsimpulsen bzw. der Behandlung der aufeinander folgenden Reaktionsräume keine Zeit für das Aufladen oder das teilweise Entladen von Ladungsspeichereinrichtungen verloren geht.

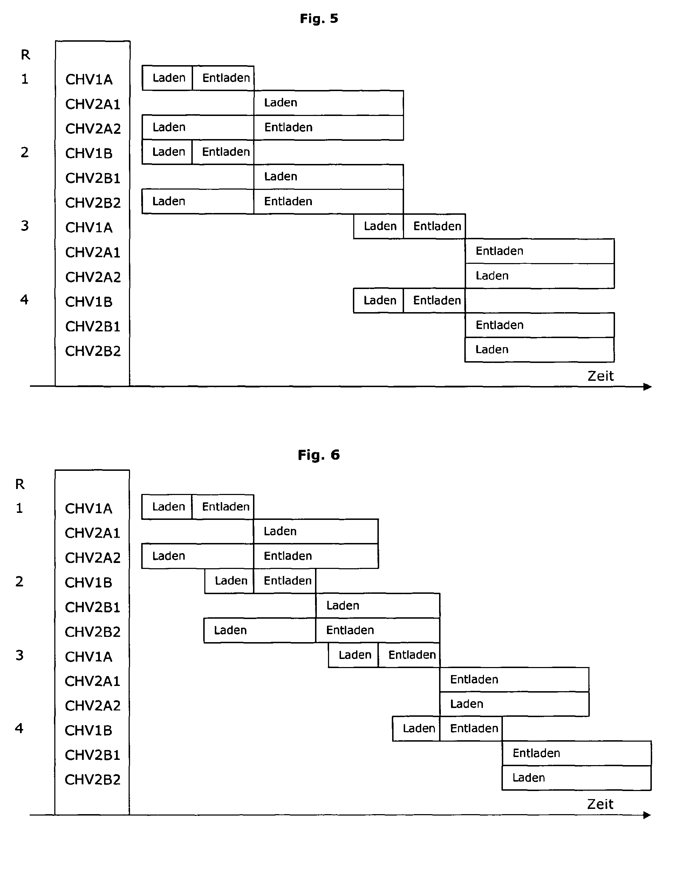

Figur 5 zeigt schematisch den zeitlichen Ablauf einer weiteren Ausführungsform des erfindungsgemäßen Verfahrens. Dieses Ausführungsbeispiel eignet sich insbesondere zur gleichzeitigen Beaufschlagung von mindestens zwei Reaktionsräumen mit jeweils mindestens zwei Spannungsimpulsen. Zu Beginn des Verfahrens werden zunächst mindestens vier Ladungsspeichereinrichtungen gleichzeitig oder nacheinander geladen. Diese vier Ladungsspeichereinrichtungen sind im vorliegenden Ausführungsbeispiel mit CHV1A, CHV2A2, CHV1 B und CHV2B2 bezeichnet. Nach Abschluss der Ladevorgänge werden zunächst zwei erste Ladungsspeichereinrichtungen CHV1A und CHV1B zumindest partiell entladen, wobei mindestens zwei Reaktionsräume, hier R1 und R2, mit jeweils einem Spannungsimpuls beaufschlagt werden. Diese beiden Reaktionsräume werden dann nach einer kurzen Pause oder ohne Unterbrechung jeweils mit einem weiteren Spannungsimpuls beaufschlagt, wobei diese Spannungsimpulse durch die zumindest partielle Entladung der zweiten Ladungsspeichereinrichtungen CHV2A2 und CHV2B2 erzeugt werden. Gleichzeitig mit der Entladung dieser zweiten Ladungsspeichereinrichtungen werden mindestens zwei weitere zweite Ladungsspeichereinrichtungen, die hier mit CHV2A1 und CHV2B1 bezeichnet sind, sowie die beiden ersten Ladungsspeichereinrichtungen CHV1A und CHV1B geladen oder teilweise entladen. Durch die anschließende zumindest partielle Entladung der beiden ersten Ladungsspeichereinrichtungen CHV1A und CHV1 B können dann dieselben (R1 und R2) oder, wie hier dargestellt, zwei weitere Reaktionsräume R3 und R4 mit jeweils einem Spannungsimpuls beaufschlagt werden. Anschließend (mit oder ohne Unterbrechung) werden dann die zuvor geladenen zweiten Ladungsspeichereinrichtungen CHV2A1 und CHV2B1 wieder entladen, während zeitlich parallel die zuvor entladenen Ladungsspeichereinrichtungen CHV2A2 und CHV2B2 wieder geladen oder teilweise entladen werden. Die zuvor beschriebenen Lade- und Entladevorgänge können dann beliebig oft wiederholt werden, so dass sehr viele Spannungsimpulse in relativ kurzer Zeit abgegeben werden können. Auf diese Weise kann auch eine Vielzahl von Reaktionsräumen in einer relativ kurzen Zeit mit Spannungsimpulsen, insbesondere Zweifachimpulsen, beaufschlagt werden.

Figur 6 zeigt schematisch den zeitlichen Ablauf einer besonderen Ausführungsform des erfindungsgemäßen Verfahrens, das im Wesentlichen der Ausführungsform gemäß Figur 5 entspricht. Im Unterschied zu dem in Figur 5 dargestellten erfindungsgemäßen Verfahren werden hier die jeweils ersten Spannungsimpulse eines Doppelspannungsimpulses nicht gleichzeitig (synchron), sondern nacheinander erzeugt. Das bedeutet, dass die Entladung der ersten Ladungsspeichereinrichtungen CHV1A und CHV1B jeweils nacheinander erfolgt, so dass die Reaktionsräume R1 und R2 bzw. R3 und R4 nicht gleichzeitig, sondern nacheinander mit den ersten Spannungsimpulsen beaufschlagt werden. Das hat zur Folge, dass auch die jeweils zweiten Spannungsimpulse eines Doppelspannungsimpulses nicht parallel, sondern leicht versetzt und überlappend abgegeben werden. Bei dieser besonderen Ausführungsform werden also im Gegensatz zur Ausführungsform gemäß Figur 5 nicht mindestens zwei Reaktionsräume gleichzeitig mit Doppelspannungsimpulsen behandelt, sondern nacheinander bzw. zeitlich leicht versetzt. Hierdurch kann zwar unter Umständen die gesamte Prozessdauer verlängert, diese Ausführungsform kann jedoch dann von Vorteil sein, wenn unerwünschte Nebeneffekte durch die Spannungsimpulse vermieden werden sollen. Ein solcher unerwünschter Nebeneffekt könnte beispielsweise eine zu starke Wärmeentwicklung sein.

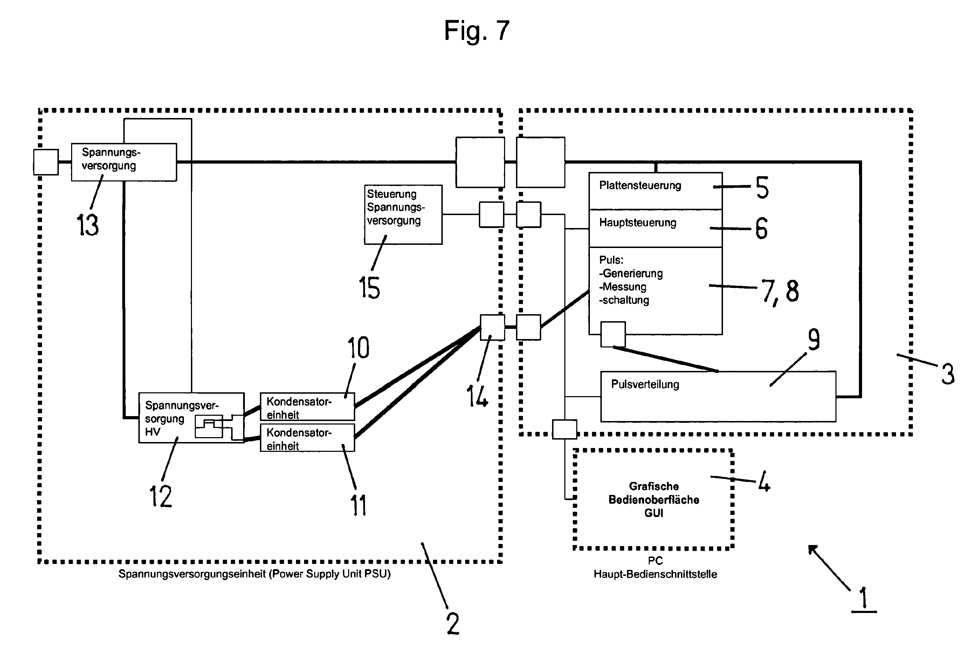

Figur 7 zeigt schematisch den Aufbau einer Ausführungsform der erfindungsgemäßen Vorrichtung zur Beaufschlagung mindestens eines Reaktionsraums mit mindestens einem Spannungsimpuls. Die erfindungsgemäße Vorrichtung 1 ist im Wesentlichen modular aufgebaut und besteht zumindest aus einem Spannungsversorgungsmodul 2, einem Impulsverteilungsmodul 3 und einem Steuermodul 4. Das Steuermodul 4 umfasst eine Steuereinheit, die einen Mikroprozessor umfasst, und kann beispielsweise ein herkömmlicher Personalcomputer sein, vorzugsweise ein Laptop. Das Impulsverteilungsmodul 3 umfasst eine Behältniskontrolleinrichtung 5, welche die Steuerung für das Behältnis beinhaltet, das den Reaktionsraum oder die Reaktionsräume umfasst. Das Impulsverteilungsmodul 3 umfasst ferner eine zentrale Steuereinrichtung 6 (Main Controller), welche die Verteilung der einzelnen Spannungsimpulse auf den Reaktionsraum oder die Reaktionsräume steuert. Das Impulsverteilungsmodul 3 weist auch eine Impulsgenerierungseinheit 7 und eine Schalteinheit 8 auf. Die Impulsgenerierungseinheit 7 umfasst Bauteile, die den Übergang aufeinander folgender Spannungsimpulse ermöglichen bzw. steuern, wie beispielsweise ein gemischter Schaltkreis, der analoge und digitale Bauteile beinhaltet. Die Schalteinheit 8 umfasst die Messschaltungen für die Spannungsimpulse und die Schalter (z.B. IGBTs oder MOSFETs) für die Entladung der Ladungsspeichereinrichtungen. Die Schalteinheit 8 ist mit der Verteilvorrichtung 9 für elektrische Spannungsimpulse gekoppelt. Die Verteilvorrichtung 9 umfasst die Schaltvorrichtungen (z.B. Relais) zur Verteilung der Spannungsimpulse und die mechanischen Einrichtungen zum Bewegen und Positionieren des Behältnisses bzw. der Behältnisse, welche die Reaktionsräume umfassen.

Das Spannungsversorgungsmodul 2 gemäß der in Figur 7 dargestellten Ausführungsform der erfindungsgemäßen Vorrichtung 1 umfasst im Wesentlichen zwei Ladungsspeichereinrichtungen 10, 11, die von einer gemeinsamen Spannungsversorgungseinrichtung 12 geladen werden können. Die Spannungsversorgungseinrichtung 12 wird mittels der Netzteilsteuerung 15 gesteuert. Es ist ferner mindestens eine hier nicht dargestellte Entladevorrichtung vorgesehen, über die die Ladungsspeichereinrichtungen 10, 11 teilweise entladen werden können. Bei der Entladevorrichtung kann es sich beispielsweise um einen Entladewiderstand handeln. Das Spannungsversorgungsmodul 2 umfasst im vorliegenden Ausführungsbeispiel ferner ein zentrales Netzteil 13, welches die Spannungsversorgung in der gesamten Vorrichtung 1 gewährleistet. Die beiden Ladungsspeichereinrichtungen 10, 11 bilden ein Speichermodul, das über eine gemeinsame Schnittstelle 14 mit dem Impulsverteilungsmodul 3 verbunden ist. Das Speichermodul, d. h. die Ladungsspeichereinrichtungen 10 und 11, sind also im vorliegenden Ausführungsbeispiel über die Impulsgenerierungseinheit 7 und die Schalteinheit 8 mit der Verteilvorrichtung 9 elektrisch gekoppelt. Dadurch, dass die beiden Ladungsspeichereinrichtungen 10, 11 ein Speichermodul bilden, in dem immer eine der beiden Ladungsspeichereinrichtungen 10, 11 durch die Spannungsversorgungseinrichtung 12 geladen wird, während die andere Ladungsspeichereinrichtung 10, 11 aufgeladen bzw. teilweise geladen und entladbar ist, steht das Speichermodul insgesamt zu jeder Zeit zur Bereitstellung eines Spannungsimpulses zur Verfügung. Durch diesen Aufbau können sehr viele Spannungsimpulse in relativ kurzer Zeit abgegeben werden, da im Vergleich zu einem einzelnen Ladungsspeicher keine Zeit für das erneute Aufladen oder das teilweise Entladen des Ladungsspeichers verloren geht. Bei den Ladungsspeichereinrichtungen 10, 11 kann es sich beispielsweise um einzelne Kondensatoren oder jeweils um ganze Kondensatorbänke handeln, die aus mehreren parallel und/oder in Reihe geschalteten Kondensatoren bestehen.

Die Erzeugung von hohen Spannungen, insbesondere von mehr als 1000 V ist auf Seiten der Elektronik mit einem bestimmten Aufwand verbunden, da hier besondere spannungssichere Bauteile zum Einsatz kommen müssen und bestimmte Abstände zwischen den Hochspannung führenden Leiterbahnen auf der Platine eingehalten werden müssen, um eine hohe Durchschlagsfestigkeit der Elektronik zu gewährleisten. Zum zweiten sind Hochspannungsnetzteile besonders aufwendig und teuer herzustellen, welche die für die Elektroporation benötigten entsprechenden Ladungs- und damit Energiemengen bei hohen Spannungen bereitstellen können. Auf der anderen Seite kann es aber für eine effiziente Elektroporation oder durch einen besonderen Aufbau der Elektroporationsvorrichtung (z.B. große Elektrodenabstände) vorteilhaft oder gar notwendig sein, solche hohen Spannungen (insbesondere >1000 V) zu erzeugen. Es stellt sich also die Aufgabe, diesen Aufwand bei der Lade-(und Entlade-) Vorrichtung für einen Energiespeicher (z.B. Kondensator oder Kondensatorbatterie) zu minimieren. Um hohe Spannungen auf der Impulsseite (Abgabe eines Impulses durch den Energiespeicher an einen Reaktionsraum) zu erzeugen, diese Spannungen auf der Ladeseite (Aufladen des Energiespeichers durch ein Netzteil, das seine Energie aus dem Stromnetz bezieht) aber zu vermeiden, kann man mehrere unabhängig verschaltbare Kondensatoren (oder andere Speichereinrichtungen) so benutzen, dass man sie beim Laden auf die Spannung U beispielsweise durch ein Netzteil alle parallel verschaltet und einen gegenüber der seriellen Verschaltung n-fach erhöhten Ladestrom und damit n-fach erhöhte Ladungsmenge n*Q in Kauf nimmt. Wenn man diese Ladeeinrichtungen nach Abschluss des Ladevorgangs seriell verschaltet, erhält man die Spannung n*U_{Lade} (U_{Lade} = Ladespannung), die man bei Impulsentladung auf den Reaktionsraum nutzen kann. Die Elektronik auf der Ladeseite und die einzelnen Kondensatoren selber brauchen nur auf eine relativ geringe Spannung U_{Lade}, z.B. auf 1000 V, ausgelegt sein, was deutlich Kosten und auch Raum spart (Überschlagsfestigkeit beispielsweise zwischen Leiterbahnen auf Leiterplatten bedeutet größere Abstände oder, wo möglich und sinnvoll, zusätzliche Isolationsmaßnahmen). Nur die sekundäre Entladeseite muss entsprechend spannungsfest für n*U_{Lade} sein.

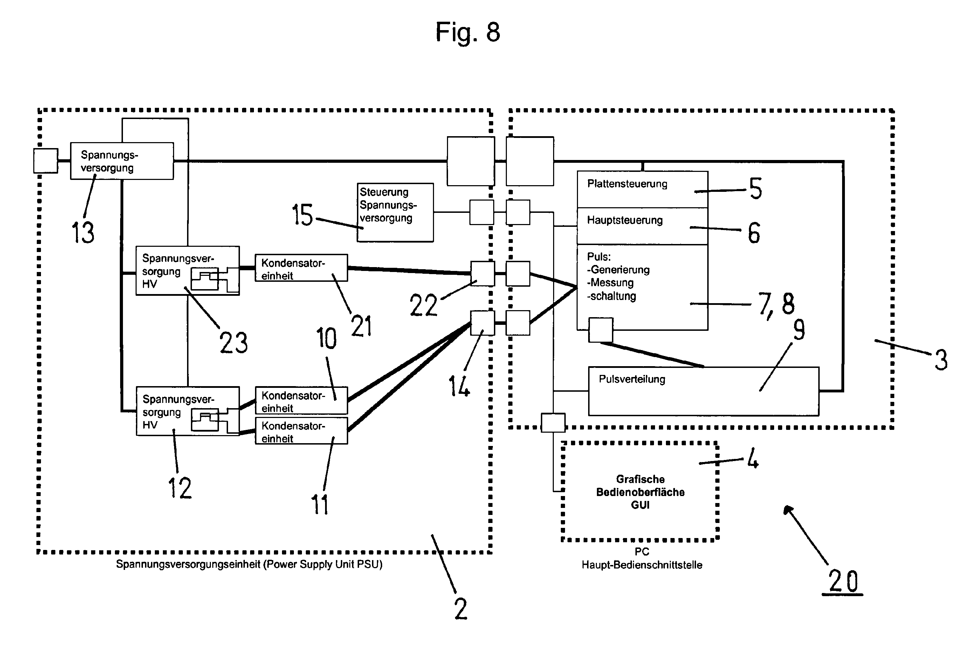

Figur 8 zeigt schematisch den Aufbau einer weiteren Ausführungsform einer erfindungsgemäßen Vorrichtung. Die Vorrichtung 20 eignet sich insbesondere zur Beaufschlagung mindestens eines Reaktionsraums mit mindestens zwei Spannungsimpulsen. Die Vorrichtung 20 entspricht hinsichtlich des modularen Aufbaus und der wesentlichen Bestandteile der Vorrichtung 1 gemäß Figur 7. Aus diesem Grund sind in Figur 8 die gleichen Bestandteile mit denselben Bezugsziffern gekennzeichnet wie in Figur 7. Die erfindungsgemäße Vorrichtung 20 gemäß Figur 8 unterscheidet sich von der Vorrichtung 1 gemäß Figur 7 dadurch, dass eine weitere Ladungsspeichereinrichtung 21 vorgesehen ist, die über eine weitere Schnittstelle 22 mit der Verteilvorrichtung 9 gekoppelt ist. Die Ladungsspeichereinrichtung 21 wird durch eine weitere Spannungsversorgungseinrichtung 23 gespeist und ermöglicht die Beaufschlagung des Reaktionsraums bzw. der Reaktionsräume mit doppelten, kurz aufeinander folgenden oder ohne Unterbrechung ineinander übergehenden Spannungsimpulsen. Diese Ausführungsform ist insbesondere dann von Vorteil, wenn Spannungsimpulse erzeugt werden sollen, die sich aus einem kurzen Spannungsimpuls, vorzugsweise mit relativ hoher Spannungsamplitude, und einem längeren Spannungsimpuls, vorzugsweise mit geringerer Spannungsamplitude, zusammensetzen. In diesem Fall kann die Ladungsspeichereinrichtung 21 einen kurzen Spannungsimpuls erzeugen, während das sich aus den Ladungsspeichereinrichtungen 10 und 11 zusammensetzende Speichermodul einen längeren Spannungsimpuls zur Verfügung stellt. Das Zusammensetzen bzw. ineinander Überleiten der einzelnen Spannungsimpulse zu einem Doppelspannungsimpuls erfolgt in der Impulsgenerierungseinheit 7. Sollen mehrere Reaktionsräume nacheinander in relativ kurzer Zeit mit derartigen Doppelspannungsimpulsen beaufschlagt werden, beispielsweise bei der Verwendung von Multiwellplatten im Hochdurchsatzverfahren, so wären die Abstände zwischen der Beaufschlagung der einzelnen Reaktionsräume bei der Verwendung eines einzelnen Kondensators für den längeren Spannungsimpuls relativ lang, da der Kondensator nach dem Entladen erst wieder aufgeladen werden müsste. Bei der erfindungsgemäßen Vorrichtung 20 kann aber immer eine der beiden Ladungsspeichereinrichtungen 10, 11 zumindest partiell entladen werden, während die andere Ladungsspeichereinrichtung 10, 11 geladen oder teilweise entladen wird, so dass sehr viele Reaktionsräume innerhalb kurzer Zeit mit Spannungsimpulsen beaufschlagt werden können. Das Aufladen bzw. teilweise Entladen der Ladungsspeichereinrichtung 21 ist dabei kein zeitlich limitierender Faktor, da die Ladungsspeichereinrichtung 21 während der länger andauernden Entladung des Speichermoduls aufgeladen oder teilweise entladen werden kann.

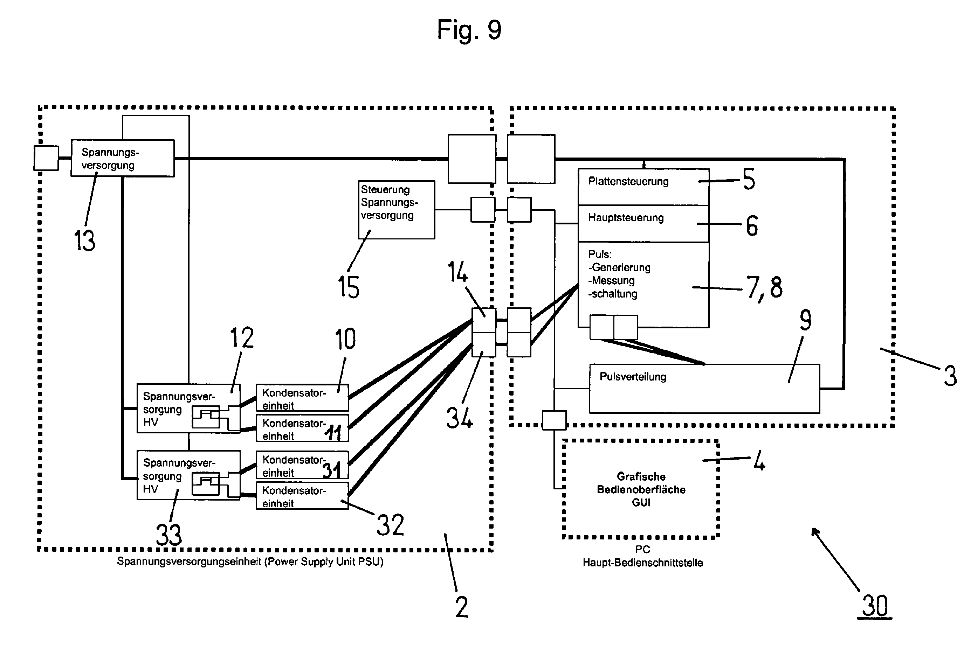

Figur 9 zeigt schematisch den Aufbau einer weiteren Ausführungsform der erfindungsgemäßen Vorrichtung, wobei sich diese Ausführungsform insbesondere zur Durchführung des erfindungsgemäßen Verfahrens zur Beaufschlagung von mindestens zwei Reaktionsräumen mit jeweils einem elektrischen Spannungsimpuls eignet. Die erfindungsgemäße Vorrichtung 30 entspricht hinsichtlich des modularen Aufbaus und der wesentlichen Bestandteile ebenfalls der Vorrichtung 1 gemäß Figur 7, wobei gleiche Bestandteile auch hier jeweils mit denselben Bezugsziffern gekennzeichnet sind. Die erfindungsgemäße Vorrichtung 30 unterscheidet sich von der Vorrichtung 1 gemäß Figur 7 dadurch, dass das Spannungsversorgungsmodul 2 zwei zusätzliche Ladungsspeichereinrichtungen 31, 32 und eine zusätzliche Spannungsversorgungseinrichtung 33 umfasst. Die Ladungsspeichereinrichtungen 31, 32 bilden ein Speichermodul, das von der gemeinsamen Spannungsversorgungseinrichtung 33 geladen werden kann und über die gemeinsame Schnittstelle 34 und die Impulsgenerierungseinheit 7 bzw. die Schalteinheit 8 mit der Verteilvorrichtung 9 elektrisch gekoppelt ist. Da die Ladungsspeichereinrichtungen 10, 11, 31, 32 insgesamt zwei Speichermodule bilden, die jeweils zu jeder Zeit entladen werden können, können durch die erfindungsgemäße Vorrichtung 30 immer zwei Reaktionsräume gleichzeitig mit jeweils einem Spannungsimpuls beaufschlagt werden. Da wie oben beschrieben zwischen zwei aufeinander folgenden Spannungsimpulsen keine Zeit für das Aufladen eines einzelnen Kondensators benötigt wird, können mittels der erfindungsgemäßen Vorrichtung 30 folglich sehr viele Reaktionsräume in relativ kurzer Zeit mit Spannungsimpulsen beaufschlagt werden. Die erfindungsgemäße Vorrichtung 30 eignet sich daher insbesondere für die Verwendung in Hochdurchsatzverfahren, bei denen sehr viele Reaktionsräume in kurzer Zeit mit jeweils einem Spannungsimpuls beaufschlagt werden müssen.

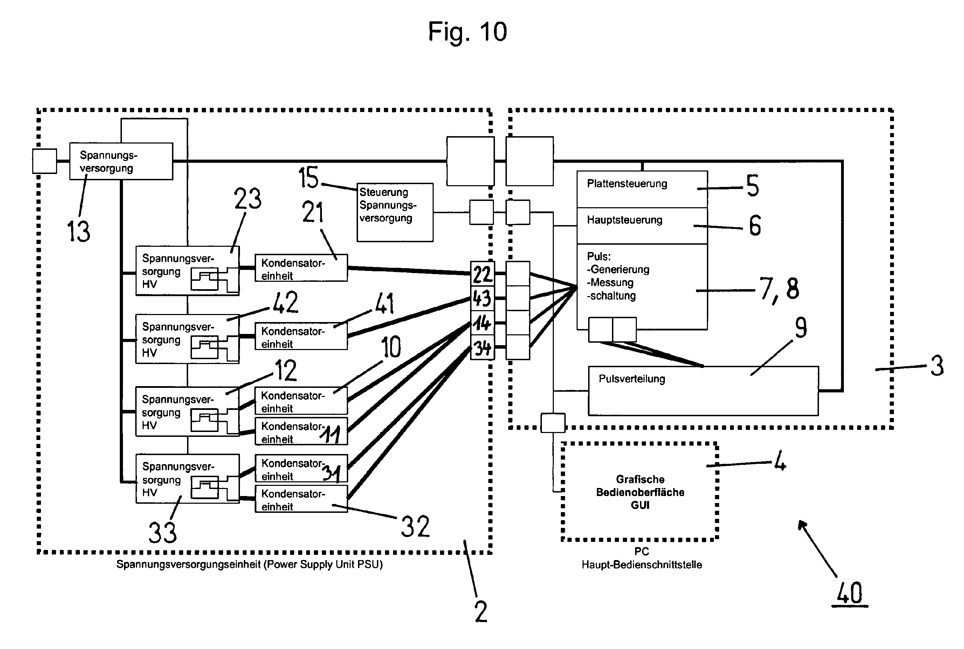

Figur 10 zeigt schematisch eine weitere Ausführungsform der erfindungsgemäßen Vorrichtung, die sich im Prinzip aus den Bestandteilen der erfindungsgemäßen Vorrichtung 20 gemäß Figur 8 und der erfindungsgemäßen Vorrichtung 30 gemäß Figur 9 zusammensetzt. Folglich sind auch hier die gleichen Bestandteile mit denselben Bezugsziffern gekennzeichnet. Die erfindungsgemäße Vorrichtung 40 umfasst innerhalb des Spannungsversorgungsmoduls 2 zusätzlich eine weitere Ladungsspeichereinrichtung 41, die durch eine eigene Spannungsversorgungseinrichtung 42 geladen werden kann und über eigene Schnittstelle 43 mit der Verteilvorrichtung 9 elektrisch gekoppelt ist. Die erfindungsgemäße Vorrichtung 40 weist also innerhalb des Spannungsversorgungsmoduls 2 insgesamt 6 Ladungsspeichereinrichtungen 10, 11, 21, 31, 32, 41 auf, die über insgesamt vier Schnittstellen 14, 22, 34, 43 mit der Verteilvorrichtung 9 gekoppelt sind. Dabei bilden jeweils zwei Ladungsspeichereinrichtungen 10 und 11 bzw. 31 und 32 insgesamt zwei Speichermodule, die wie oben beschrieben zu jeder Zeit entladen werden können. Die Ladungsspeichereinrichtungen 10, 11, 21, 31, 32, 41 werden über insgesamt vier Spannungsversorgungseinrichtungen 12, 23, 33, 42 geladen, wobei jedem Speichermodul jeweils eine Spannungsversorgungseinrichtung 12, 33 zugeordnet ist. Alternativ wäre es auch möglich, alle Ladungsspeichereinrichtungen 10, 11, 21, 31, 32, 41 durch eine Spannungsversorgungseinrichtung zu laden, beispielsweise unmittelbar durch eine der Spannungsversorgungseinrichtungen 12, 23, 33 oder 42. Die erfindungsgemäße Vorrichtung 40 ist besonders vorteilhaft einsetzbar in Hochdurchsatzverfahren, in denen viele Reaktionsräume in kurzer Zeit mit doppelten Spannungsimpulsen beaufschlagt werden müssen. Dabei können mittels der erfindungsgemäßen Vorrichtung 40 immer zwei Reaktionsräume gleichzeitig mit Spannungsimpulsen beaufschlagt werden. So können beispielsweise die Ladungsspeichereinrichtungen 21 und 41 gleichzeitig jeweils einen kurzen Spannungsimpuls mit relativ hoher Spannungsamplitude abgeben, der dann jeweils ohne Unterbrechung in einen längeren Spannungsimpuls mit geringerer Spannungsamplitude übergeht. Die längeren Spannungsimpulse können durch die beiden Speichermodule zur Verfügung gestellt werden, wobei immer eine der beiden Ladungsspeichereinrichtungen 10, 11 bzw. 31, 32 eines Speichermoduls entladen werden kann. Das Zusammenbauen des Doppelspannungsimpulses erfolgt in diesem Ausführungsbeispiel in der Impulsgenerierungseinheit 7.

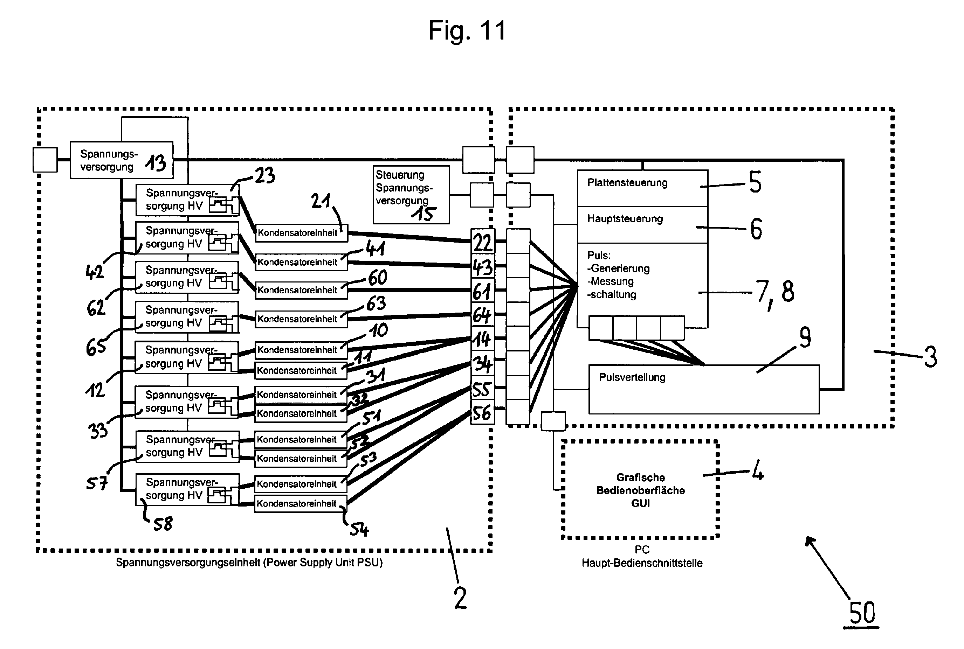

Figur 11 zeigt eine weitere besondere Ausführungsform der erfindungsgemäßen Vorrichtung, die in ihrem Aufbau im Prinzip der Vorrichtung 40 gemäß Figur 10 entspricht. Im Unterschied zur Vorrichtung 40 gemäß Figur 10 liegt bei der erfindungsgemäßen Vorrichtung 50 gemäß Figur 11 eine Verdopplung der Komponenten des Spannungsversorgungsmoduls 2 vor. Folglich können mit der erfindungsgemäßen Vorrichtung 50 vier Reaktionsräume gleichzeitig mit Spannungsimpulsen beaufschlagt werden. Die erfindungsgemäße Vorrichtung 50 umfasst zusätzlich zwei weitere Speichermodule, die durch die Ladungsspeichereinrichtungen 51 und 52 bzw. 53 und 54 gebildet werden, wobei die beiden zusätzlichen Speichermodule jeweils über eine Schnittstelle 55 bzw. 56 elektrisch mit der Verteilvorrichtung 9 gekoppelt sind. Jedes zusätzliche Speichermodul wird dabei über eine eigene Spannungsversorgungseinrichtung 57 bzw. 58 geladen. Um die gleichzeitige Beaufschlagung von vier Reaktionsräumen mit doppelten Spannungsimpulsen zu ermöglichen, sind ferner zwei weitere Ladungsspeichereinrichtungen 60 und 63 vorgesehen, die jeweils über eine separate Schnittstelle 61 bzw. 64 mit der Verteilvorrichtung 9 gekoppelt sind und jeweils über eine eigene Spannungsversorgungseinrichtung 62 bzw. 65 geladen werden können. Mittels der erfindungsgemäßen Vorrichtung 50 kann also im Vergleich zur Vorrichtung 40 gemäß Figur 10 entweder die Zeit zur Beaufschlagung der gleichen Anzahl von Reaktionsräumen deutlich reduziert oder aber die Anzahl der zu behandelnden Reaktionsräume deutlich erhöht werden. Selbstverständlich kann die Anzahl der Ladungsspeichereinrichtungen bzw. Speichermodule weiter erhöht werden, um noch mehr Reaktionsräume gleichzeitig mit Spannungsimpulsen beaufschlagen zu können.

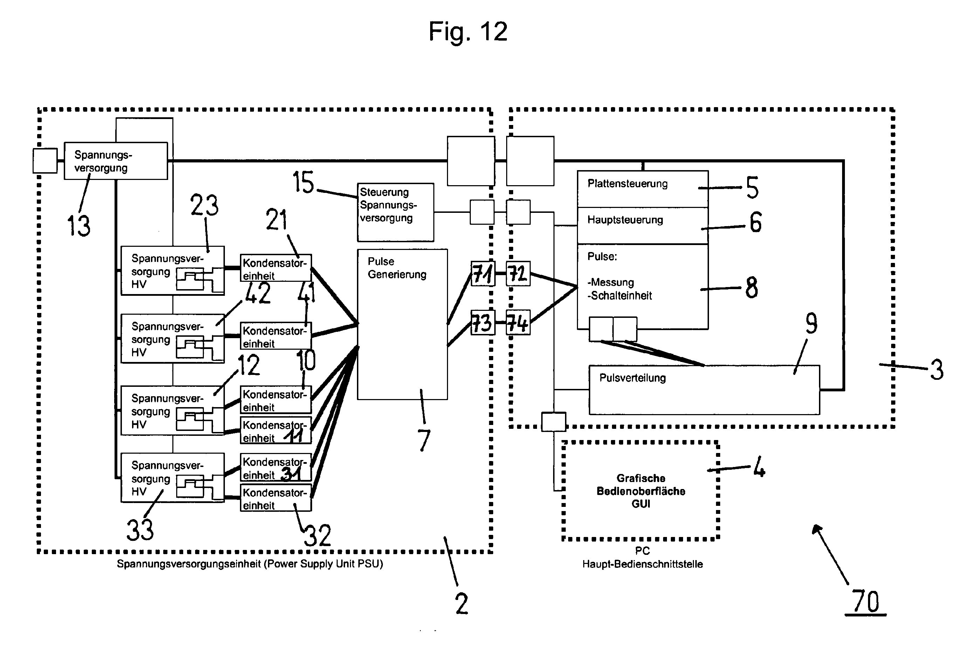

Figur 12 zeigt schematisch eine besondere Ausführungsform der erfindungsgemäßen Vorrichtung, die im Wesentlichen der erfindungsgemäßen Vorrichtung 40 gemäß Figur 10 entspricht. Daher sind auch hier die gleichen Bestandteile mit denselben Bezugsziffern gekennzeichnet. Die Vorrichtung 70 gemäß Figur 12 unterscheidet sich von der Ausführungsform gemäß Figur 10 dadurch, dass hier die Impulsgenerierungseinheit 7 innerhalb des Spannungsversorgungsmoduls 2 angeordnet ist. Die Impulsgenerierungseinheit 7 ist also in dieser Ausführungsform nicht als Einheit mit der Schalteinheit 8 innerhalb des Impulsverteilungsmoduls 3 angeordnet. Die Ladungsspeichereinrichtungen 10, 11, 21, 31, 32, 41 sind daher bei dieser Ausführungsform unmittelbar mit der Impulsregenerierungseinheit 7 gekoppelt. Dies hat den Vorteil, dass hier der Doppelspannungsimpuls, mit dem die einzelnen Reaktionsräume beaufschlagt werden, bereits im Spannungsversorgungsmodul 2 zusammengebaut werden kann, bevor der Spannungsimpuls über die Schalteinheit 8 an die Verteilvorrichtung 9 weitergegeben wird. Innerhalb der Impulsregenerierungseinheit 7 wird dabei der Doppelspannungsimpuls aus dem ersten Spannungsimpuls, der durch die Ladungsspeichereinrichtung 21 oder 41 zur Verfügung gestellt wird, und dem zweiten Spannungsimpuls, der durch die Ladungsspeichereinrichtungen 10 bzw. 11 oder 31 bzw. 32 zur Verfügung gestellt wird, generiert. Der komplette Doppelspannungsimpuls wird dann über die Schnittstellen 71 und 72 bzw. 73 und 74 an die Schalteinheit 8 weitergegeben. Die Pulsgenerierungseinheit 7 kann beispielsweise einen gemischten Schaltkreis umfassen, der analoge und digitale Bauteile beinhaltet, über den der erste und der zweite Spannungsimpuls zu einem Doppelspannungsimpuls zusammengeschaltet werden kann. Besonders vorteilhaft an dieser Ausführung ist die Reduzierung der zwischen den Geräteeinheiten zu führenden Hochspannungskabel. Da diese Kabel und die dazu gehörige Verbindungstechnik aufwendig sind, kann dadurch die Gesamtkomplexität gesenkt und die Zuverlässigkeit insbesondere für den Hochdurchsatzbetrieb gesteigert werden.

### Bezugszeichenliste:

- 1: Vorrichtung
- 2: Spannungsversorgungsmodul
- 3: Impulsverteilungsmodul
- 4: Steuermodul
- 5: Behältniskontrolleinrichtung
- 6: Steuereinrichtung (Main Controller)
- 7: Impulsgenerierungseinheit
- 8: Schalteinheit
- 9: Verteilvorrichtung
- 10: Ladungsspeichereinrichtung
- 11: Ladungsspeichereinrichtung
- 12: Spannungsversorgungseinrichtung
- 13: Netzteil
- 14: Schnittstelle
- 15: Netzteilsteuerung
- 20: Vorrichtung
- 21: Ladungsspeichereinrichtung
- 22: Schnittstelle
- 23: Spannungsversorgungseinrichtung
- 30: Vorrichtung
- 31: Ladungsspeichereinrichtung
- 32: Ladungsspeichereinrichtung
- 33: Spannungsversorgungseinrichtung
- 34: Schnittstelle
- 40: Vorrichtung
- 41: Ladungsspeichereinrichtung
- 42: Spannungsversorgungseinrichtung
- 43: Schnittstelle
- 50: Vorrichtung
- 51: Ladungsspeichereinrichtung
- 52: Ladungsspeichereinrichtung

- 53: Ladungsspeichereinrichtung
- 54: Ladungsspeichereinrichtung
- 55: Schnittstelle
- 56: Schnittstelle
- 57: Spannungsversorgungseinrichtung
- 58: Spannungsversorgungseinrichtung
- 60: Ladungsspeichereinrichtung
- 61: Schnittstelle
- 62: Spannungsversorgungseinrichtung
- 63: Ladungsspeichereinrichtung
- 64: Schnittstelle
- 65: Spannungsversorgungseinrichtung
- 70: Vorrichtung
- 71: Schnittstelle
- 72: Schnittstelle
- 73: Schnittstelle
- 74: Schnittstelle

## Patentansprüche

1. Verfahren zur Beaufschlagung mindestens eines Reaktionsraums mit mindestens einem elektrischen Spannungsimpuls durch zumindest partielles Entladen mindestens einer Ladungsspeichereinrichtung zum Speichern elektrischer Ladungen, welches die folgenden Schritte umfasst:
a) Beaufschlagen mindestens eines Reaktionsraums mit mindestens einem Spannungsimpuls durch zumindest partielles Entladen einer ersten Ladungsspeichereinrichtung und gleichzeitig Laden oder teilweises Entladen einer zweiten Ladungsspeichereinrichtung;
b) Beaufschlagen desselben Reaktionsraums oder eines weiteren Reaktionsraums mit mindestens einem Spannungsimpuls durch zumindest partielles Entladen der zweiten Ladungsspeichereinrichtung und gleichzeitig Laden oder teilweises Entladen der während Schritt a) entladenen ersten Ladungsspeichereinrichtung.

2. Verfahren nach Anspruch 1, **gekennzeichnet durch** die folgenden Schritte:
a) Beaufschlagen von mindestens zwei Reaktionsräumen mit jeweils mindestens einem Spannungsimpuls **durch** zumindest partielles Entladen von zwei ersten Ladungsspeichereinrichtungen und gleichzeitig Laden oder teilweises Entladen von zwei zweiten Ladungsspeichereinrichtungen;
b) Beaufschlagen derselben Reaktionsräume oder von zwei weiteren Reaktionsräumen mit jeweils mindestens einem Spannungsimpuls **durch** zumindest partielles Entladen der beiden zweiten Ladungsspeichereinrichtungen und gleichzeitig Laden oder teilweises Entladen der beiden während Schritt a) zumindest partiell entladenen ersten Ladungsspeichereinrichtungen.

3. Verfahren nach Anspruch 1, **gekennzeichnet durch** die folgenden Schritte:
a) Beaufschlagen mindestens eines Reaktionsraums mit mindestens einem Spannungsimpuls **durch** zumindest partielles Entladen einer ersten Ladungsspeichereinrichtung;
b) Beaufschlagen des Reaktionsraums mit mindestens einem weiteren Spannungsimpuls **durch** zumindest partielles Entladen einer zweiten Ladungsspeichereinrichtung und gleichzeitig Laden oder teilweises Entladen einer weiteren zweiten Ladungsspeichereinrichtung sowie der während Schritt a) entladenen ersten Ladungsspeichereinrichtung;
c) Beaufschlagen eines anderen oder desselben Reaktionsraums mit mindestens einem Spannungsimpuls **durch** zumindest partielles Entladen der während Schritt a) zumindest partiell entladenen und während Schritt b) wieder geladenen oder teilweise entladenen ersten Ladungsspeichereinrichtung;
d) Beaufschlagen des Reaktionsraums aus Schritt c) mit jeweils mindestens einem weiteren Spannungsimpuls **durch** zumindest partielles Entladen der während Schritt b) geladenen oder teilweises entladenen weiteren zweiten Ladungsspeichereinrichtung und gleichzeitig Laden oder teilweises Entladen der während Schritt b) zumindest partiell entladenen zweiten Ladungsspeichereinrichtung sowie der während Schritt c) zumindest partiell entladenen ersten Ladungsspeichereinrichtung.

4. Verfahren nach Anspruch 1, **gekennzeichnet durch** die folgenden Schritte:
a) Beaufschlagen von mindestens zwei Reaktionsräumen mit jeweils mindestens einem Spannungsimpuls **durch** zumindest partielles Entladen von zwei ersten Ladungsspeichereinrichtungen;
b) Beaufschlagen der beiden Reaktionsräume aus Schritt a) mit jeweils mindestens einem weiteren Spannungsimpuls **durch** zumindest partielles Entladen von zwei zweiten Ladungsspeichereinrichtungen und gleichzeitig Laden oder teilweises Entladen von zwei weiteren zweiten Ladungsspeichereinrichtungen sowie der beiden während Schritt a) zumindest partiell entladenen ersten Ladungsspeichereinrichtungen;
c) Beaufschlagen derselben Reaktionsräume oder von zwei weiteren Reaktionsräumen mit jeweils mindestens einem Spannungsimpuls **durch** zumindest partielles Entladen der beiden während Schritt a) zumindest partiell entladenen und während Schritt b) wieder geladenen oder teilweise entladenen ersten Ladungsspeichereinrichtungen;
d) Beaufschlagen der beiden Reaktionsräume aus Schritt c) mit jeweils mindestens einem weiteren Spannungsimpuls **durch** zumindest partielles Entladen der beiden während Schritt b) geladenen oder teilweise entladenen weiteren zweiten Ladungsspeichereinrichtungen und gleichzeitig Laden oder teilweises Entladen der beiden während Schritt b) zumindest partiell entladenen zweiten Ladungsspeichereinrichtungen sowie der beiden während Schritt c) zumindest partiell entladenen ersten Ladungsspeichereinrichtungen.

5. Verfahren nach einem der Ansprüche 1 bis 4, **dadurch gekennzeichnet, dass** jeweils mindestens zwei Ladungsspeichereinrichtungen ein Speichermodul bilden, wobei immer eine der Ladungsspeichereinrichtungen in der Anwendung zumindest partiell entladen wird, während die andere Ladungsspeichereinrichtung geladen oder teilweise entladen wird, so dass das Speichermodul insgesamt zu jeder Zeit die für den nächsten Spannungsimpuls benötigte Spannung bereithält und zumindest partiell entladen werden kann.

6. Verfahren nach einem der Ansprüche 1 bis 5, **dadurch gekennzeichnet, dass** Reaktionsräume nacheinander beaufschlagt werden, die räumlich möglichst weit voneinander entfernt sind.

7. Verfahren zur Beaufschlagung von mindestens zwei Reaktionsräumen mit jeweils mindestens einem elektrischen Spannungsimpuls durch Entladen mindestens einer Ladungsspeichereinrichtung zum Speichern elektrischer Ladungen, **dadurch gekennzeichnet, dass** das Entladen der Ladungsspeichereinrichtung derart gesteuert wird, dass mindestens zwei Reaktionsräume nacheinander mit Spannungsimpulsen unterschiedlicher Spannungen beaufschlagt werden, bei denen die nach der Beaufschlagung des ersten Reaktionsraums in der Ladungsspeichereinrichtung verbleibende Spannung der Spannung des darauf folgend abzugebenden Spannungsimpulses möglichst nahe kommt.

8. Vorrichtung (1, 20, 30, 40, 50, 70) zur Beaufschlagung mindestens eines Reaktionsraums mit mindestens einem Spannungsimpuls, welche mindestens zwei elektrische Ladungsspeichereinrichtungen (10, 11) zum Speichern von elektrischen Ladungen und mindestens eine Spannungsversorgungseinrichtung (12) zum Laden der Ladungsspeichereinrichtungen (10, 11) und/oder mindestens eine Entladevorrichtung zum teilweisen Entladen der Ladungsspeichereinrichtungen (10, 11) umfasst, **dadurch gekennzeichnet, dass** zumindest zwei der Ladungsspeichereinrichtungen (10, 11) ein gemeinsames Speichermodul bilden, das zu jeder Zeit entladbar ist.

9. Vorrichtung nach Anspruch 8, **dadurch gekennzeichnet, dass** die mindestens zwei Ladungsspeichereinrichtungen (10, 11) über eine gemeinsame Schnittstelle (14) mit einer Impulsgenerierungseinheit (7), einer Schalteinheit (8) und/oder einer Verteilvorrichtung (9) für elektrische Spannungsimpulse elektrisch gekoppelt sind, und dass mindestens eine weitere Ladungsspeichereinrichtung (21) vorgesehen ist, die vorzugsweise über mindestens eine weitere Schnittstelle (22) mit der Impulsgenerierungseinheit (7), der Schalteinheit (8) und/oder der Verteilvorrichtung (9) für elektrische Spannungsimpulse elektrisch gekoppelt ist.

10. Vorrichtung nach Anspruch 9, **dadurch gekennzeichnet, dass** die mindestens eine weitere Ladungsspeichereinrichtung (21) mit einer Spannungsversorgungseinrichtung (23) elektrisch gekoppelt ist und dass zumindest die mindestens zwei Ladungsspeichereinrichtungen (10, 11) mit einer gemeinsamen Spannungsversorgungseinrichtung (12) elektrisch gekoppelt sind.

11. Vorrichtung nach einem der Ansprüche 8 bis 10, **dadurch gekennzeichnet, dass** mindestens vier Ladungsspeichereinrichtungen (10, 11, 31, 32) vorgesehen sind, und dass jeweils mindestens zwei der Ladungsspeichereinrichtungen (10, 11, 31, 32) ein Speichermodul bilden, das jeweils über eine gemeinsame Schnittstelle (14, 34) mit der Impulsgenerierungseinheit (7), der Schalteinheit (8) und/oder der Verteilvorrichtung (9) für elektrische Spannungsimpulse elektrisch gekoppelt ist.

12. Vorrichtung nach Anspruch 11, **dadurch gekennzeichnet, dass** mindestens zwei Spannungsversorgungseinrichtungen (12, 33) vorgesehen sind, wobei jede Spannungsversorgungseinrichtung (12, 33) mit jeweils einem Speichermodul elektrisch gekoppelt ist.

13. Vorrichtung nach Anspruch 12, **dadurch gekennzeichnet, dass** die Ladungsspeichereinrichtungen (10, 11, 21, 31, 32, 41) über mindestens vier Schnittstellen (14, 22, 34, 43) mit der Impulsgenerierungseinheit (7), der Schalteinheit (8) und/oder der Verteilvorrichtung (9) elektrisch gekoppelt sind.

14. Vorrichtung nach Anspruch 12 oder 13, **dadurch gekennzeichnet, dass** zumindest zwei der Ladungsspeichereinrichtungen (21, 41) mit jeweils einer Spannungsversorgungseinrichtung (23, 42) elektrisch gekoppelt sind und dass mindestens zwei Speichermodule vorgesehen sind, die jeweils mindestens zwei Ladungsspeichereinrichtungen (10, 11, 31, 32) umfassen, wobei zumindest zwei der Speichermodule jeweils mit einer gemeinsamen Spannungsversorgungseinrichtung (12, 33) elektrisch gekoppelt sind.

15. Vorrichtung nach einem der Ansprüche 8 bis 14, **dadurch gekennzeichnet, dass** zumindest eine Ladungsspeichereinrichtung (10, 11, 21, 31, 32, 41) mit einer Impulsgenerierungseinheit (7) elektrisch gekoppelt ist, welche ihrerseits, vorzugsweise über eine Schalteinheit (8), mit einer Verteilvorrichtung (9) für elektrische Spannungsimpulse elektrisch gekoppelt ist.
